(19) Europäisches Patentamt
European Patent Office
Office européen des brevets

(11) **EP 3 173 405 A1**

(12) **EUROPÄISCHE PATENTANMELDUNG**

(43) Veröffentlichungstag:
**31.05.2017 Patentblatt 2017/22**

(51) Int Cl.:
***C07C 303/16*** *(2006.01)*

(21) Anmeldenummer: **15197066.2**

(22) Anmeldetag: **30.11.2015**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **Evonik Degussa GmbH**
**45128 Essen (DE)**

(72) Erfinder:
• **FONFE, Benjamin**
**60318 Frankfurt (DE)**
• **DÜRR, Nadine**
**64665 Alsbach (DE)**
• **JAKOB, Harald**
**63594 Hasselroth (DE)**
• **CHEUNG, Chiu Kee**
**63755 Alzenau (DE)**
• **DÖRFLEIN, Andreas**
**63538 Großkrotzenburg (DE)**
• **FUSS, Sebastian**
**36103 Flieden (DE)**

(54) **VERFAHREN ZUR HERSTELLUNG EINER ALKANSULFONSÄURE**

(57) Die vorliegende Erfindung betrifft ein Verfahren zur Herstellung einer Alkansulfonsäure durch Oxidation eines Alkylmercaptans, eines Dialkyldisulfids und/oder eines Dialkylpolysulfids mit drei bis neun Schwefelatomen mit einem Oxidationsmittel, wobei zusätzliches Oxidationsmittel in die Oxidation eingespeist wird, sofern im Reaktionsaustrag aus der Oxidation noch nicht oxidiertes Alkylmercaptan und/oder nicht oxidiertes Dialkyldisulfid und/oder mindestens ein Zwischenprodukt aus der Oxidation des Dialkyldisulfids und/oder des Dialkylpolysulfids vorhanden ist, und eine entsprechende Vorrichtung zur Durchführung von Oxidationsreaktionen.

**EP 3 173 405 A1**

**Beschreibung**

Die vorliegende Erfindung betrifft ein Verfahren zur Herstellung einer Alkansulfonsäure durch Oxidation eines Alkylmercaptans, eines Dialkyldisulfids und/oder eines Dialkylpolysulfids mit drei bis neun Schwefelatomen mit einem Oxidationsmittel, wobei zusätzliches Oxidationsmittel in die Oxidation eingespeist wird, sofern im Reaktionsaustrag aus der Oxidation noch nicht oxidiertes Alkylmercaptan und/oder nicht oxidiertes Dialkyldisulfid und/oder mindestens ein Zwischenprodukt aus der Oxidation des Dialkyldisulfids und/oder des Dialkylpolysulfids vorhanden ist, und eine entsprechende Vorrichtung zur Durchführung von Oxidationsreaktionen.

Alkansulfonsäuren sind die organischen Derivate der Schwefelsäure, von der sie sich strukturell durch den Ersatz einer Hydroxyl-Gruppe durch einen organischen Rest unterscheiden. Die allgemeine Strukturformel der Alkansulfonsäuren lautet daher $R-SO_3-H$, wobei R einen organischen Rest bezeichnet, beispielsweise Alkyl oder Aryl. In Abhängigkeit dieses organischen Rests wird zwischen aliphatischen oder aromatischen Sulfonsäuren unterschieden. Die freien Sulfonsäuren sind in der Regel farblose und hygroskopische Substanzen deren Säurestärke denen der anorganischen Säuren entspricht. Mit einem pKs-Wert von -5,5 ist Trifluormethansulfonsäure sogar eine der stärksten bekannten Säuren und gehört daher zur Gruppe der sogenannten Supersäuren. Im Gegensatz zu den Sulfatsalzen von Quecksilber, Blei und Silber sind die entsprechenden Sulfonate sehr gut wasserlöslich.

Aus dem Stand der Technik, beispielsweise den Offenlegungsschriften WO 98/34914 A1, US 2,433,395, US 2,433,396, US 2,498,318, US 2,502,618, US 2,505,910, US 2,697,722, US 2,727,920, WO 00/31027 A1 und CN 101648892 A, ist eine Vielzahl von Verfahren zur großtechnischen Herstellung von Alkansulfonsäuren durch Oxidation von Alkylmercaptan, Dialkyldisulfiden und Dialkypolysulfiden bekannt. Geht das betreffende Verfahren von einem Alkylmercaptan aus, wird dieses Mercaptan zum entsprechenden Dialkyldisulfid oxidiert, welches im Anschluss weiter zur Alkansulfonsäure oxidiert wird. Laut der in der Literatur vertretenen Auffassung verläuft die Oxidation von Dialkyldisulfiden R-S-S-R zu den entsprechenden Alkansulfonsäuren über die Zwischenstufen eines Alkansulfoxid-S-alkylethers R-S-SO-R, eines Alkansulfonsäure-S-alkylesters $R-S-SO_2-R$, eines Alkansulfoxid-S-alkylthiosulfonats $R-SO-SO_2-R$ und eines Dialkyldisulfons $R-SO_2-SO_2-R$, wobei letzteres schließlich zur gewünschten Alkansulfonsäure $R-SO_3-H$ hydrolysiert wird. Bei der Herstellung von Alkansulfonsäuren durch Oxidation von Dialkylpolysulfiden mit drei oder mehr Schwefelatomen werden die Bildung von Alkyldisulfiden und Alkylmercaptanen und deren anschließende Oxidation entsprechend der Oxidation von Dialkyldisulfiden und Alkylmercaptanen in der Literatur diskutiert.

Für eine möglichst hohe Ausbeute an Alkansulfonsäure wird eine vollständige Umsetzung der jeweiligen Ausgangsstoffe zur Alkansulfonsäure angestrebt. Im Stand der Technik wird daher meistens mit einem Überschuss an Salpetersäure als Oxidationsmittel gearbeitet, wie beispielsweise in der WO 00/31027 beschrieben. Durch den großen Anteil Salpetersäure in der Reaktionsmischung werden allerdings auch beträchtliche Mengen Wasser in die Reaktion eingebracht, das anschließend energie- und kostenintensiv von dem gewünschten Produkt abgetrennt werden muss. Ein weiterer Nachteil dieses Verfahrens besteht in der Bildung großer Mengen von gesundheitsschädlichen und umweltgefährdenden Stickstoffoxiden, von denen Distickstoffoxid $N_2O$ auch als sogenanntes Treibhausgas gilt. Zur Vermeidung der Freisetzung dieser Stickstoffoxide müssen daher entsprechende Maßnahmen getroffen werden, die ebenfalls kosten- und energieintensiv sind. Dies macht die Oxidation von Alkylmercaptanen, Dialkyldisulfiden und/oder Dialkypolysulfiden mit einem Überschuss Salpetersäure zu Alkansulfonsäuren in der Gesamtbetrachtung wirtschaftlich unattraktiv.

Alternativ wird daher die Oxidation von Alkylmercaptanen, Dialkyldisulfiden und/oder Dialkypolysulfiden zu Alkansulfonsäuren auch mit Luft und einer unterstöchiometrischen Menge Salpetersäure als Katalysator durchgeführt. Bei dieser Verfahrensweise muss allerdings sichergestellt werden, dass ausreichend Luft, mit Sauerstoff angereicherte Luft oder sogar reiner Sauerstoff als Oxidationsmittel in die Reaktion eingespeist wird, um eine möglichst vollständige Oxidation der Ausgangsstoffe und Zwischenprodukte zur Alkansulfonsäure sicherzustellen. Ist dies nicht gewährleistet, werden die Ausgangsstoffe und/oder die Oxidationszwischenprodukte nicht oder nur unvollständig zur gewünschten Alkansulfonsäure oxidiert. In diesem Fall müssen große Mengen an nicht umgesetzten Ausgangsstoffen und/oder Oxidationszwischenprodukten aus der Rohalkansulfonsäure abgetrennt und in die Reaktion zurückgeführt werden.

Die daraus resultierende mangelnde ökonomische Effizienz stellt jedoch noch ein vergleichsweise kleines Problem dar. Problematisch ist vielmehr, dass bei einer nicht vollständigen Umsetzung der Oxidationszwischenprodukte zur Alkansulfonsäure elementarer Schwefel ausfällt. Dieser Niederschlag tritt vor allem bei Temperaturen von mehr als 90°C auf und stellt daher insbesondere für eine bei Temperaturen von deutlich mehr als 90°C durchgeführte Destillation des Reaktionsgemisches ein ernsthaftes Problem dar. Denn bereits in geringen Mengen setzt dieser Schwefelniederschlag die vom Destillat durchströmten Bauteile der Destillationskolonne, wie Leitungen und Kolonnenköpfe, zu, was zu Betriebsstillständen und somit zwangsläufig zu Produktionsausfällen führt. Es wird vermutet, dass dieser Schwefelniederschlag auf die Zersetzung von Schwefel enthaltenden Oxidationsprodukten, ggf. begleitet von einer Synproportionierung von Schwefelatomen unterschiedlicher Oxidationsstufen, unter Freisetzung von elementarem Schwefel zurückzuführen ist.

Typischerweise wird eine Produktionsanlage nicht immer mit derselben Auslastung betrieben. Vielmehr schwankt die Auslastung einer Anlage in Abhängigkeit von der Nachfrage und der Menge der eingesetzten Ausgangsmaterialien. Ferner können auch variierende Prozessparameter wie Temperatur und Druck einen Einfluss auf den Grad der Umsetzung zur Alkansulfonsäure haben.

Um auch bei unterschiedlichen Auslastungen einer Produktionsanlage und variierenden Prozessparametern immer eine gleichbleibend hohe Umsetzung zur Alkansulfonsäure und die Vermeidung eines Schwefelniederschlags zu gewährleisten, bedarf es daher einer geeigneten Prozessführung.

Diese Aufgabe wird erfindungsgemäß dadurch gelöst, indem eine nicht oder nicht vollständig zur Alkansulfonsäure umgesetzte schwefelhaltige Ausgangsverbindung, vorzugsweise ein Alkylmercaptan, Dialkyldisulfid und/oder Dialkylpolysulfid mit drei bis neun Schwefelatomen, und/oder ein Oxidationszwischenprodukt aus der Oxidation eines Dialkyldisulfids und/oder Dialkylpolysulfids zur Alkansulfonsäure im Reaktionsaustrag aus der Oxidationsreaktion bestimmt werden und bei ihrer Anwesenheit im Reaktionsaustrag aus der Oxidationsreaktion zusätzliches Oxidationsmittel in die Oxidationsreaktion zugeführt wird. Auf diese Weise wird die Oxidationsreaktion zur gewünschten Alkansulfonsäure vervollständigt.

Gegenstand der vorliegenden Erfindung ist daher ein Verfahren zur Herstellung einer Alkansulfonsäure, umfassend die Schritte

a) Oxidieren mindestens einer schwefelhaltigen Ausgangsverbindung mit einem Oxidationsmittel zur korrespondierenden Alkansulfonsäure, wobei die schwefelhaltige Ausgangsverbindung ausgewählt ist aus der Gruppe bestehend aus einem Alkylmercaptan, einem Dialkyldisulfid und einem Dialkylpolysulfid mit drei bis neun Schwefelatomen,

b) Bestimmen mindestens einer schwefelhaltige Ausgangsverbindung und/oder mindestens eines Oxidationszwischenproduktes aus der Oxidation der schwefelhaltigen Ausgangsverbindung zur korrespondierenden Alkansulfonsäure im Reaktionsaustrag von Schritt a), und

c) Zuführen von zusätzlichem Oxidationsmittel in Schritt a) zur Vervollständigung der Oxidationsreaktion, wenn in Schritt b) eine schwefelhaltige Ausgangsverbindung und/oder ein Oxidationszwischenprodukt aus der Oxidation der schwefelhaltigen Ausgangsverbindung zur korrespondierenden Alkansulfonsäure bestimmt wurden.

Im Rahmen der vorliegenden Erfindung werden die Begriffe Alkylmercaptan, Dialkyldisulfid und Dialkylpolysulfid entsprechend dem allgemeinen Wissen des Fachmanns verwendet. Daher entsprechen ein Alkylmercaptan der allgemeinen Formel R-SH, ein Dialkyldisulfid der allgemeinen Formel R-S-S-R und ein Dialkylpolysulfid der allgemeinen Formel $R\text{-}S_n\text{-}R$ mit n = 3 bis 9, wobei R eine Alkylgruppe ist. Typischerweise weisen die Alkylgruppen der im Rahmen der vorliegenden Erfindung eingesetzten Alkylmercaptane, Dialkyldisulfide oder Dialkylpolysulfide 1 bis 12 Kohlenstoffe auf. Hinsichtlich der Größe und Struktur dieser Alkylgruppen, beispielsweise bezüglich zusätzlicher funktioneller Gruppen, unterliegt das erfindungsgemäße Verfahren keinen Beschränkungen, sofern gewährleistet ist, dass die funktionellen Gruppen der Alkylgruppen unter den in der Oxidationsreaktion vorherrschenden Reaktionsbedingungen selber nicht reaktiv sind und die betreffenden schwefelhaltigen Verbindungen in dem eingesetzten Lösungsmittel zumindest teilweise löslich sind. Das Lösungsmittel kann beim erfindungsgemäßen Verfahren auch die herzustellende Alkansulfonsäure selber sein. Daher können die Alkylmercaptane, Dialkyldisulfide bzw. Dialkylpolysulfide lineare oder verzweigte Alkylgruppen aufweisen, bevorzugt linear, mit jeweils 1 bis 12 Kohlenstoffatomen, bevorzugt 1 bis 6 oder 1 bis 4 Kohlenstoffatomen. Besonders bevorzugt sind die Alkylgruppen ausgewählt aus der Gruppe bestehend aus Methyl, Ethyl, n-Propyl, iso-Propyl, n-Butyl, iso-Butyl und tert-Butyl.

Die aus wirtschaftlicher Sicht bedeutendste Alkansulfonsäure ist die Methansulfonsäure, da ihre Metallsalze in großen Mengen als Elektrolyte in methansulfonsauren Elektrolysebädern in der Herstellung von Schaltkreisen für die Elektronikindustrie eingesetzt werden. Zudem findet Methansulfonsäure zunehmend Verwendung als Bestandteil von Reinigungsmitteln, da es auf Grund seiner Farb- und Geruchslosigkeit leicht in Reinigungslösungen integriert werden kann. Eine weiteres wichtiges neues Anwendungsgebiet für Methansulfonsäure ist der Einsatz beim Ölbohren: Die Erdöl führenden Schichten, die durch Bohrlöcher zugänglich gemacht werden, geben das Öl oftmals nur in einem begrenzten Masse oder überhaupt nicht frei. Zur Erzielung einer verstärkten Freisetzung des Öls werden daher die Erdöl führenden Gesteinsschichten mit Methansulfonsäure aufgeweicht. Darüber hinaus findet Methansulfonsäure auch Anwendung als Katalysator in einer Vielzahl von organischen Reaktionen, wie Alkylierungen, Veresterungen, Polymerisationen oder Heterozyklen-Synthesen, oder in der Bildung von Säureadditionssalzen mit basischen Wirkstoffen.

Bevorzugt ist daher die schwefelhaltige Ausgangsverbindung des erfindungsgemäßen Verfahrens Methylmercaptan, Dimethyldisulfid und/oder Dimethylpolysulfid mit drei bis neun Schwefelatomen und die herzustellende Alkansulfonsäure Methansulfonsäure.

Wenn die schwefelhaltige Ausgangsverbindung Dimethyldisulfid und/oder Dimethylpolysulfid ist, geht dem erfindungsgemäßen Verfahren eine separate Herstellung von Dimethyldisulfid und/oder Dimethylpolysulfid voran, bevorzugt durch Umsetzung von Methylmercaptan mit elementarem Schwefel. Der dabei gebildete Schwefelwasserstoff wird in das betreffende Verfahren zur Herstellung von Methylmercaptan zurückgeführt.

Im erfindungsgemäßen Verfahren können die schwefelhaltigen Ausgangsverbindungen einzeln oder im Gemisch der Oxidationsreaktion von Schritt a) zugeführt werden. Beim Einsatz von schwefelhaltigen Ausgangsverbindungen mit zwei Alkylgruppen sind Dialkyldisulfide und Dialkylpolysulfide mit identischen Alkylgruppen bevorzugt; diese Verbindungen werden im Rahmen der vorliegenden Erfindung auch als symmetrische Dialkyldisulfide bzw. symmetrische Dialkylpolysulfide bezeichnet. Die Durchführung des erfindungsgemäßen Verfahrens mit symmetrischen Dialkyldisulfiden bzw. Dialkylpolysulfiden führt zu einer einzigen Alkansulfonsäure, was die Aufarbeitung des Produktgemisches signifikant erleichtert. Im Gegensatz dazu führt der Einsatz unsymmetrischer Dialkyldisulfide und/oder unsymmetrischer Dialkylpolysulfide zu einem Gemisch von Alkansulfonsäuren, welches gegebenenfalls nur mit erheblichem Aufwand in die einzelnen Alkansulfonsäuren getrennt werden kann. Vorzugsweise werden im erfindungsgemäßen Verfahren daher symmetrische Dialkyldisulfide oder Dialkylpolysulfide eingesetzt. Beim Einsatz unterschiedlicher Ausgangsverbindungen, beispielsweise einem Alkylmercaptan und einem Dialkyldisulfid, sind daher die Alkylgruppen dieser Verbindungen vorzugsweise ebenfalls identisch.

Bei Verwendung eines Alkylmercaptan als Ausgangsverbindung wird das Alkylmercaptan zunächst zum entsprechenden Dialkyldisulfid oxidiert. Das so erhaltene bzw. das direkt als Ausgangsverbindung eingesetzte Dialkyldisulfid, und dementsprechend auch ein Dialkylpolysulfid, unterliegt bei der Oxidation zur Alkansulfonsäure einer Reihe von einzelnen Oxidationsschritten, durch die eine schrittweise Übertragung von Sauerstoffatomen auf die Schwefelatome erfolgt, was zur Bildung von Derivaten des betreffenden Dialkyldisulfids bzw. Dialkylpolysulfids mit mindestens einem an einem Schwefel gebundenen Sauerstoff führt. Im Falle eines Dialkyldisulfids R-S-S-R verläuft diese schrittweise Oxidation über die in der Literatur diskutierten Zwischenprodukte eines Alkansulfoxid-S-alkylesters R-S-SO-R, eines Alkansulfonsäure-S-alkylesters $R\text{-}S\text{-}SO_2\text{-}R$ oder eines Dialkyldisulfoxids R-SO-SO-R oder eines Alkansulfoxid-S-alkylthiosulfonats $R\text{-}SO\text{-}SO_2\text{-}R$ und eines Dialkyldisulfons $R\text{-}SO_2\text{-}SO_2\text{-}R$. Abschließend wird in Anwesenheit von Wasser ein Äquivalent des Dialkyldisulfons in zwei Äquivalente der entsprechenden Alkansulfonsäure überführt.

Während der Oxidation eines Dialkydisulfids zur Alkansulfonsäure liegen sämtliche dieser Zwischenprodukte entweder gleichzeitig oder nacheinander in dem Reaktionsgemisch von Schritt a) vor.

Liegt das für die Oxidation einer schwefelhaltigen Ausgangsverbindung zur korrespondierenden Alkansulfonsäure erforderliche Oxidationsmittel im Unterschuss bezogen auf die eingesetzte Menge der schwefelhaltigen Ausgangsverbindung vor, werden sowohl die schwefelhaltige Ausgangsverbindung als auch die Zwischenprodukte der Oxidationsreaktion entweder gar nicht oder nicht vollständig zur gewünschten Alkansulfonsäure oxidiert. Dies wirkt sich unmittelbar negativ auf die Ausbeute für die gewünschte Alkansulfonsäure aus. In einer aufwändigen Aufreinigung müssen dann die mindestens eine nicht umgesetzte Ausgangsverbindung und die Oxidationszwischenprodukte von der Alkansulfonsäure abgetrennt und in die Oxidationsreaktion zurückgeführt werden. Dies ist mit erheblichen zusätzlichen Investitions- und Betriebskosten verbunden.

Um eine möglichst hohe Ausbeute für die gewünschte Alkansulfonsäure zu erreichen und gleichzeitig eine Akkumulation von Oxidationszwischenprodukten im Reaktionsgemisch zu vermeiden, ist es daher zwingend erforderlich, in regelmäßigen Zeitabständen und vorzugsweise kontinuierlich schwefelhaltige Ausgangsverbindungen und/oder Oxidationszwischenprodukte im Reaktionsaustrag aus Schritt a) zu bestimmen (Schritt b)) und bei ihrer Anwesenheit zusätzliches Oxidationsmittel in Schritt a) zur Vervollständigung der Oxidationsreaktion zuzuführen (Schritt c)). Auf Basis der Bestimmung der schwefelhaltigen Ausgangsverbindungen und/oder Oxidationszwischenprodukte im Reaktionsaustrag aus Schritt a) wird die zur Vervollständigung der Oxidation in Schritt a) erforderliche Menge zusätzlichen Oxidationsmittels ermittelt. Vorzugsweise wird mindestens die Menge zusätzlichen Oxidationsmittels in Schritt a) zugeführt, die zur Vervollständigung der Oxidationsreaktion erforderlich ist. Insbesondere ist die Menge des in Schritt c) zugeführten zusätzlichen Oxidationsmittels größer als die zur Vervollständigung der Oxidation in Schritt a) erforderliche Menge zusätzlichen Oxidationsmittels. Dadurch wird eine Vervollständigung der Oxidationsreaktion sichergestellt.

Die großtechnische Trennung eines Produktgemisches aus der Herstellung von Alkansulfonsäuren durch Oxidation schwefelhaltiger Ausgangsverbindungen erfolgt in der Regel durch eine Destillation, was mit erheblichen Investitions- und Energiekosten verbunden ist. Insbesondere die Energiekosten sind erheblich, wenn man die hohen Temperaturen berücksichtigt, die für ein Überkopf-Destillieren der betreffenden Alkansulfonsäure erforderlich sind. Bereits der einfachste Vertreter der Alkansulfonsäuren, die Methansulfonsäure, weist einen Siedepunkt von 167°C bei 13 hP auf.

Temperaturen von mehr 90°C sind allerdings hoch problematisch, wenn die Oxidation schwefelhaltiger Ausgangsverbindungen zur korrespondierenden Alkansulfonsäure nicht oder nicht vollständig verlaufen ist. Denn für entsprechende Reaktions- bzw. Produktgemische wird bei Temperaturen von mehr als 90°C ein Niederschlag von elementarem Schwefel beobachtet. Bereits geringe Mengen ausgefallenen Schwefels können sowohl die vom Reaktor wegführenden Rohrleitungen als auch die Kolonnenköpfe und Leitungen von Destillationseinrichtungen verstopfen, was zwangsläufig zu Anlagenstillständen und somit zu Produktionsstillständen führt. Es wird vermutet, dass der Niederschlag von Schwefel auf die Zersetzung des Oxidationszwischenproduktes Alkansulfonsäure-S-alkylester und ggf. eine Synproportionierung der Schwefelatome unterschiedlicher Oxidationsstufen zurückzuführen ist, der sich vermutlich bei einer nicht vollständig verlaufenen Oxidation von schwefelhaltigen Ausgangsverbindungen zur gewünschten Alkansulfonsäuren im Reaktionsgemisch akkumuliert. Zur Vermeidung von Anlagen- und Produktionsstillständen ist es daher erforderlich, die Anwesenheit von Alkansulfonsäure-S-alkylestern im Reaktionsaustrag von Schritt a) in zumindest regelmäßigen Zeitabständigen und bevorzugt kontinuierlich zu bestimmen und bei Anwesenheit von Alkansulfonsäure-S-alkylester im Reaktionsaustrag von Schritt a) zusätzliches Oxidationsmittel zur Vervollständigung der Oxidation in Schritt a) zuzuführen.

In einer Ausführungsform des erfindungsgemäßen Verfahrens wird daher in Schritt b) ein Alkansulfonsäure-S-alkylester als Oxidationszwischenprodukt bestimmt.

Da im erfindungsgemäßen Verfahren vorzugsweise Methylmercaptan, Dimethyldisulfid und/oder Dimethylpolysulfide als schwefelhaltige Ausgangsverbindungen eingesetzt werden, ist das in

Schritt b) des erfindungsgemäßen Verfahrens bestimmte Oxidationszwischenprodukt bevorzugt Methansulfonsäure-S-methylester bzw. Methylmethanthiosulfonat (MMTS).

Im Rahmen der vorliegenden Erfindung hat sich gezeigt, dass Oxidationszwischenprodukte aus der Herstellung von Alkansulfonsäuren, insbesondere oxidierte Dialkyldisulfide oder Dialkypolysulfide mit einem oder mehreren Sauerstoffatomen an einem Schwefelatom mit Hilfe der UV-Vis-Spektroskopie verlässlich und präzise bestimmbar sind. Insbesondere Alkansulfonsäure-S-alkylester sind auf Grund ihrer stark polaren Ester-Gruppe für die Bestimmung mittels UV-Vis-Spektroskopie geeignet. Im Gegensatz dazu lassen sich Alkansulfonsäuren praktisch nicht mit Hilfe der UV-Vis-Spektroskopie bestimmen. Folglich ermöglicht die UV-Vis-Spektroskopie eine verlässliche Bestimmung von Zwischenstufen aus der Oxidation von Dialkyldisulfiden bzw. Dialkylpolysulfiden zur Alkansulfonsäure insbesondere in einer die korrespondierende Alkansulfonsäure enthaltenden Matrix, bei der die anwesende Alkansulfonsäure die Bestimmung der Alkansulfonsäure-S-alkylester nicht beeinträchtigt.

In einer Ausführungsform des erfindungsgemäßen Verfahrens erfolgt die Bestimmung des Oxidationszwischenprodukts, insbesondere des Alkansulfonsäure-S-alkylesters, mittels UV-VIS-Spektroskopie.

Der Begriff UV-Vis-Spektroskopie wird gemäß dem allgemeinen Fachwissen des Fachmanns verwendet und bezeichnet in seiner allgemeinsten Form eine Methode, die elektromagnetische Wellen des ultravioletten (UV) und des sichtbaren (englisch visible, VIS) Lichts nutzt zur Bestimmung von Verbindungen, die im UV- und/oder im Vis-Bereich Licht absorbieren. Ein für diese Bestimmung verwendetes UV-Vis-Spektrometer enthält eine Lichtquelle mit einer Blende, ein Prisma zur Monochromatisierung, eine Küvette mit einer die zu bestimmende Verbindung enthaltenden Probe, einen Detektor und einen Verstärker. Ein Einstrahl-UVNis-Spektrometer enthält nur eine Küvette mit einer die zu bestimmende Verbindung enthaltenden Probe. Die Funktionsweise eines Einstrahl-UV/Vis-Spektrometer lässt sich wie folgt beschreiben: Die Lichtquelle strahlt ultraviolettes, sichtbares und nahinfrarotes Licht im Wellenlängenbereich von 200 nm bis 1100 nm aus. Im Monochromator wird die zur Messung ausgewählte Wellenlänge des Lichts selektiert, und das monochromatische Licht tritt durch die Küvette mit der betreffenden Probe. Der Detektor misst die Intensität des durch den durchstrahlten Körper hindurchgetretenen Lichts. Bei dieser Messung kann allerdings auch das Lösungsmittel bzw., falls der Bestimmung eine chromatographische Trennung vorangeht, auch der Eluent der Probe Licht absorbieren oder Licht an der Küvette reflektiert bzw. gestreut werden. In sämtlichen Fällen würde dies zu einer Abschwächung der Intensität des durch den durchstrahlten Körpers hindurchtretenden Lichts führen, was die Qualität der Bestimmung deutlich beeinflussen würde. Daher wird vor jeder Messung der betreffenden Probe in einem Einstrahlspektrometer zunächst eine Vergleichsmessung mit einer Küvette, die nur das Lösungsmittel der Probe und, falls der Bestimmung eine chromatographische Trennung vorangeht, auch den Eluenten der Probe enthält, durchgeführt. Der für die Vergleichsmessung erhaltene Wert wird gespeichert und anschließend wird die Probe vermessen. Die für die Vergleichsmessung und die Probe gemessenen Intensitäten werden im Verstärker verglichen, und der Verstärker passt dann die Intensität des Lichtstrahls für die Messlösung über eine Blende an die Intensität der Vergleichsmessung an. Bei einem Zweistrahl-Spektrometer fällt das monochomatische Licht auf einen Sektorspiegel, der abwechselnd das Licht durch die Messlösung und die Vergleichslösung fallen lässt. Beide Lösungen befinden sich in Küvetten. Die zwei Lichtstrahlen werden im Detektor empfangen und die Intensitäten im Verstärker verglichen. Der Verstärker passt dann die Intensität des Lichtstrahls aus der Vergleichslösung durch Einfahren einer Kammblende der Intensität des Lichtstrahls aus der Messlösung an.

Die vom Detektor gemessene abgeschwächte Intensität des durch den durchstrahlten Körper hindurchgetretenen Lichts der Wellenlänge $\lambda$ wird auch als Extinktion $E_\lambda$ bezeichnet und ist ein Maß für die Absorption der zu bestimmenden Verbindung für das Licht der Wellenlänge $\lambda$. Die Schwächung der Strahlungsintensität mit der Weglänge beim Durchgang durch eine absorbierende Substanz wird durch das Lambert-beersche Gesetz beschrieben: Die Extinktion $E_\lambda$ (Absorbanz der Substanz für Licht der Wellenlänge $\lambda$) ist gegeben durch

$$E_\lambda = \lg (I_0 / I) = \varepsilon_\lambda \cdot c \cdot d,$$

mit

I = Intensität des durch die Probe hindurchgelassenen (transmittierten) Lichts (Einheit: $W \cdot m^{-2}$)

$I_0$ = Intensität des einfallenden (eingestrahlten) Lichts (Einheit: $W \cdot m^{-2}$)

D = Schichtdicke des durchstrahlten Körpers (Einheit: m)

c = Stoffmengenkonzentration der absorbierenden Verbindung in der Flüssigkeit (Einheit: $mol^{-1}$)

$\varepsilon_\lambda$ = dekadischer Extinktionskoeffizient (oft auch als spektraler Absorptions-koeffizient bezeichnet) bei der Wellenlänge $\lambda$. Dieser ist eine für die absorbierende Substanz spezifische Größe und kann unter anderem vom pH-Wert oder vom Lösungsmittel abhängen. Bei einer Konzentrationsangabe in Mol wird $\varepsilon_\lambda$ als dekadischer molarer Extinktions-koeffizient angegeben, beispielsweise in der Einheit $m^2 \cdot mol^{-1}$.

Die Bestimmung von Methansulfonsäure-S-methylester erfolgt bei einer Wellenlänge von 210 nm mit einer Bandbreite von +/- 5 nm und einer Messdauer von 300 ms.

Im Gegensatz zu den Alkansulfonsäure-S-alkylestern führt die UV-Vis-Detektion bei schwefelhaltigen Ausgangsverbindungen, insbesondere Dialkyldisulfiden und Dialkylpolysulfiden, nicht zu befriedigenden Ergebnissen. Beispielsweise erlaubt die Verwendung von eines Hochleistungsflüssigkeits-Chromatographen (HPLC, high performance liquid chromatography) mit einem UV-Detektor die Bestimmung von Dimethyldisulfid nur mit einem Fehler von ungefähr 10% bei 1 Gew.-% Dimethyldisulfid. Auch die Analyse mittels der sogenannten Headspace-Gaschromatographie (GC, gas chromatography), bei welcher nur der Dampfraum über der Probe analysiert und somit der Analyt kaum beeinflusst wird, eignet sich nicht für die Bestimmung von Dialkyldisulfiden oder Dialkylpolysulfiden. Denn diese Methode erlaubt lediglich bei synthetischen Proben gute Ergebnisse, führt aber bei der Bestimmung von Dialkyldisulfiden in einer Realprobenmatrix nicht zu befriedigenden Ergebnissen. Auch bei einem Wechsel von der Hochleistungsflüssigkeits-Chromatographie zur Ionenchromatographie (IC, ion chromatography) werden keine Verbesserungen erzielt, denn Dialkyldisulfide wie Dimethyldisulfid lassen sich mit einem UV-Detektor nicht mit der erforderlichen Genauigkeit bestimmen. Dies trifft auch für die Verwendung eines Leitfähigkeitsdetektors und eines Brechungsindex-Detektors zur Bestimmung von Dialkyldisulfiden zu. Im Routinebetrieb sind mit der NMR-Analytik (nuclear magentic resonance, Kernspinresonanz) Nachweisgrenzen von ca. 0,1 Gew.-% realisierbar. Diese Nachweisgrenze ist jedoch für die Anwendung in der Bestimmung von Dialkyldiuslfiden und Dialkylpolysulfiden in einer Matrix, insbesondere in einer Alkansulfonsäure-Matrix, zu hoch. Ein weiterer Nachteil der Kernspinresonanz besteht darin, dass NMR-Spektrometer hochkomplizierte, kosten- und wartungsintensive Analysengeräte sind, deren Einsatz in unmittelbarer Nähe zu einer Produktionsanlage nicht praktikabel ist. Zudem ist der Aufwand zur Erzielung einer niedrigeren Nachweisgrenze mittels Kernspinresonanz schlichtweg zu groß für eine Anwendung als Routinemessgerät. Denselben Limitierungen bzgl. der Nachweisgrenze unterliegt auch die NIR-Analytik (near infrared, Nahinfrarot-Spektroskopie). Im Rahmen der vorliegenden Erfindung hat sich gezeigt, dass die gepulste amperometrische Detektion für die Bestimmung schwefelhaltiger Ausgangsverbindung in Schritt b) geeignet ist.

In einer Ausführungsform des erfindungsgemäßen Verfahrens erfolgt daher die Bestimmung der schwefelhaltigen Ausgangsverbindung in Schritt b) mittels gepulster amperometrische Detektion.

Die amperometrische Detektion bzw. Amperometrie ist eine elektrochemische Methode zur quantitativen Bestimmung von chemischen Verbindungen. Grundvoraussetzung für die Anwendbarkeit der Amperometrie ist, dass die zu bestimmenden chemischen Verbindungen leicht oxidierbare oder reduzierbare Substanzen sind. Der Aufbau der Detektorzelle in welcher die Messung stattfindet beruht auf einer sogenannten potentiostatischen Messanordnung. Daher besitzt eine entsprechende Detektorzelle drei Elektroden: eine Arbeitselektrode, die zur Verfolgung der elektrochemischen Vorgänge dient, eine Hilfselektrode, die den Strom aus der Oxidation bzw. Reduktion transportiert, und eine Referenzelektrode, die hochohmig geschaltet ist und somit für eine gleichmäßige Spannung an der Arbeitselektrode sorgt; der Stromfluss wird somit zwischen Arbeits- und Hilfselektrode gemessen. Liegt an der Arbeitselektrode das für eine Oxidation oder Reduktion der betreffenden organischen Verbindung erforderliche Potential an, wird als Folge dieser elektrochemischen Reaktion ein Signalstrom gemessen. Der gemessene Elektrolysestrom wird anschließend verstärkt und als Funktion der Zeit in einem Chromatogramm dargestellt. Da der Elektrolysestrom direkt proportional ist zur Konzentration der in der Elektrolysereaktion umgesetzten organischen Verbindungen, erlaubt die amperometrische Detektion die Bestimmung unbekannter Konzentrationen einer spezifischen organischen Verbindung mit Hilfe einer zuvor erstellten Kalibrierfunktion. Im Rahmen der vorliegenden Erfindung wird eine amperometrische Detektion, bei der nur das für die Durchführung der Elektrolysereaktion der organischen Verbindung erforderliche Potential an die Arbeitselektrode angelegt wird, als amperometrische Detektion bei konstantem Potential bezeichnet.

Für die der vorliegenden Erfindung zugrundeliegenden Aufgabestellung hat sich allerdings gezeigt, dass eine amperometrische Bestimmung bei konstantem Potential noch keine reproduzierbaren Ergebnisse für die Bestimmung eines Dialkyldisulfids in einer Alkansulfonsäure erlaubt: In der Regel ist der bei einer Wiederholung einer vorangegangenen Messung ermittelte Wert für den Elektrolysestrom deutlich niedriger als bei der ersten Messung, beispielsweise kann der Messwert über einen Zeitraum von einer Stunde um über 20% abnehmen. Zurückgeführt wird dies zum einen auf die (teilweise) Belegung der Arbeitselektrode mit mindestens einem Adsorbat und auf das Auftreten kapazitiver Ströme. Bei dem mindestens einen Adsorbat kann es sich um ein Produkt der elektrochemischen Reaktion, die an der Arbeitselektrode stattgefunden hat, handeln. Das Auftreten kapazitiver Ströme wird vermutlich dadurch hervorgerufen,

6

dass die Ausbildung einer Diffusionsschicht an der Arbeitselektrode bzw. ihre Ausbreitung in die Lösung hinein behindert wird, was auf die Umverteilung von elektroaktiven Spezies in der Detektorzelle auf Grund typischer Konvektionsvorgänge zurückgeführt wird.

Erfindungsgemäß werden die erforderliche Genauigkeit und Reproduzierbarkeit der amperometrischen Detektion dadurch erzielt, indem sie gepulst wird. Im Rahmen der vorliegenden Erfindung wird unter einer sogenannten gepulsten amperometrischen Detektion eine amperometrische Detektion verstanden, bei der die an der Arbeitselektrode zur Durchführung der Elektrolyse anliegende Spannung in periodischen Zeitabständen durch mindestens einen rechteckigen Puls überlagert wird. Bei diesem rechteckigen Puls kann es sich sowohl um ein anodisches als auch ein kathodisches Potential sowie eine Mischung aus beidem handeln. Durch diese Technik wird die Arbeitselektrode nicht nur von den an ihrer Oberfläche haftenden Adsorbaten gesäubert, sondern sie wird auch für die nächsten Bestimmungen konditioniert. Diese Konditionierung verbessert die Ausbildung der Diffusionsschicht an der Arbeitselektrode bzw. deren Ausbreitung in die Lösung hinein, was ebenfalls zu einer erheblichen Verbesserung der Genauigkeit und Reproduzierbarkeit der gepulsten amperometrischen Detektion gegenüber einer "einfachen" amperometrischen Detektion bei konstanter Spannung beiträgt.

Der bei einer gepulsten amperometrischen Detektion gemessene Elektrolysestrom wird nicht nur durch die zu bestimmende schwefelhaltige Ausgangsverbindung vorgegeben, insbesondere ob es sich um ein Alkylmercaptan oder ein Dialkyldisulfid bzw. Dialkylpolysulfid handelt, sondern auch von ihrer Struktur und ihrer Konzentration. Um eine zuverlässige und präzise Bestimmung der eingesetzten schwefelhaltigen Verbindung, beispielsweise von Dimethyldisulfid, gewährleisten zu können, muss daher vor der Bestimmung eine entsprechende Kalibrierkurve für die betreffende schwefelhaltige Ausgangsverbindung erstellt werden. Die Erstellung einer Kalibrierkurve wird typischerweise so durchgeführt, dass zunächst der Elektrolysestrom für Lösungen unterschiedlicher Konzentrationen der in Schritt a) eingesetzten, also bekannten, schwefelhaltigen Ausgangsverbindung, beispielsweise von Dimethyldisulfid, gemessen wird. Diese Messwerte werden dann mit den betreffenden Konzentrationen des Dimethyldisulfids korreliert und in einer Kalibrierfunktion für Dimethyldisulfid wiedergegeben. Anschließend wird für eine Probe mit einer unbekannten Konzentration des Dimethyldisulfids (für das zuvor die entsprechende Kalibrierfunktion erstellt wurde) der Elektrolysestrom mittels gepulster amperometrischer Detektion gemessen. Durch Vergleich mit der Kalibrierfunktion für Dimethyldisulfid kann anhand des gemessenen Elektrolysestroms die Konzentrationen für dieses Dialkyldisulfid in der betreffenden Probe ermittelt werden. Analog zu dieser Vorgehensweise erfolgt auch die Erstellung entsprechender Kalibrierfunktion für andere schwefelhaltige Ausgangsverbindungen. Wird in Schritt a) des erfindungsgemäßen Verfahrens ein Alkylmercaptan als schwefelhaltige Ausgangsverbindung eingesetzt, umfasst der Ausdruck schwefelhaltige Ausgangsverbindung in diesem Fall sowohl das Alkylmercaptan als auch das durch eine erste Oxidation des Alkylmercaptans erhaltene Dialkyldisulfid, welches dann die eigentliche schwefelhaltige Ausgangsverbindung für die Oxidation in Schritt a) des erfindungsgemäßen Verfahrens darstellt. In diesem Fall werden Kalibrierfunktionen sowohl für das anfangs eingesetzte Alkylmercaptan als auch für das aus der Oxidation des Alkylmercaptans unmittelbar erhaltene Dialkyldisulfids erstellt.

Vorzugsweise umfasst der Schritt b) des erfindungsgemäßen Verfahrens daher die Einzelschritte

b1) Messen des Elektrolysestroms für eine schwefelhaltige Ausgangsverbindung mittels gepulster amperometrischer Detektion, und

b2) Ermitteln der Menge und/oder der Konzentration der zu bestimmenden schwefelhaltigen Ausgangsverbindung durch Vergleich mit einer zuvor für die zu bestimmende schwefelhaltige Ausgangsverbindung erstellten Kalibrierfunktion.

Ein notwendiges Kriterium, damit eine Schwefel enthaltende organische Verbindung wie ein Dialkyldisulfid mit Hilfe der amperometrischen Detektion bestimmt werden kann, ist das Vorliegen von mindestens einem freien Elektronenpaar am Schwefelatom. Denn nur Verbindungen mit einem freien Elektronenpaar können an Elektrodenoberflächen adsorbiert werden und sind daher elektroaktive Spezies. Im Gegensatz dazu stellen organische Verbindungen ohne freie Elektronenpaare nicht-elektroaktive Spezies dar. Denn diese Verbindungen können auf Grund fehlender Elektronenpaare nicht an Elektrodenoberflächen adsorbiert und daher auch nicht mittels amperometrischer Detektion bestimmt werden.

Vorzugsweise wird daher die gepulste amperometrische Detektion im oxidativen Modus durchgeführt.

Die Durchführung der gepulsten amperometrischen Detektion im oxidativen Modus zur Bestimmung von schwefelhaltigen Verbindungen hat den Vorteil, dass die betreffende, an der Elektrodenoberfläche adsorbierte schwefelhaltige Ausgangsverbindung entweder in einem Schritt oder schrittweise zu einer Verbindung oxidiert wird, die nicht elektroaktiv ist. Beispielsweise wird Dimethyldisulfid direkt zu einer Derivat mit mindestens einem an einem Schwefel kovalent gebundenen Sauerstoff oxidiert. Das Endprodukt dieser Oxidation ist ein Dimethylsulfon, das in Anwesenheit von Wasser zur Methansulfonsäure hydrolysiert wird. Ein anderes Beispiel ist Methylmercaptan, das zunächst zum Dimethyldisulfid und anschließend über die vorstehend beschriebenen Oxidationszwischenprodukte zum Dimethylsulfon oxidiert wird, das schließlich in Anwesenheit von Wasser zur Methansulfonsäure hydrolysiert wird. Die in jedem Fall als Endprodukt der elektrolytischen Oxidation vorliegende Alkansulfonsäure ist nicht elektroaktiv. Denn das Schwefelatom dieser Verbindung verfügt über kein freies Elektronenpaar mit dem es an der Elektrodenoberfläche adsorbieren könnte. Daher können sich auch keine Adsorbate von Alkansulfonsäure an der Elektrodenoberfläche aufbauen, die entweder die laufende Messung oder spätere Messungen beeinflussen könnten. In vorteilhafter Weise trägt dies zur Genauigkeit des erfindungsgemäßen Verfahrens bei. Im Rahmen der vorliegenden Erfindung wird daher unter einer gepulsten amperometrischen Detektion im oxidativen Modus auch eine Detektion verstanden, bei der die an der Elektrodenoberfläche adsorbierte Komponente Alkylmercaptan, Dialkyldisulfid und/oder Dialkylpolysulfid entweder vollständig oder zumindest nahezu vollständig in oxidierte Spezies, wie beispielsweise $R^1$-$SO_2$-$SO_2$-$R^1$ mit $R^1$ einem Alkylrest, überführt werden.

Für die Durchführung von gepulsten amperometrischen Detektionen sind prinzipiell alle bekannten unterschiedlichen Typen von Kohlenstoff- und Edelmetall-Elektroden geeignet. Typischerweise werden für reduktive Bestimmungen Edelmetall-Elektroden, wie Gold-, Silber- und Goldamalgam-Elektroden, sowie Quecksilberfilm-Elektroden eingesetzt (siehe J. Frank, Chimia 1981, 35, 24, P. T. Kissinger, C.S. Brunett, K. Bratin, J.R. Rice, Spec. Publ. (U.S.) 1979, 519, 705 und S. Yao, A. Meyer, G. Henze, Fresenius J. Anal. Chem. 1991, 339, 207).

Im Rahmen des Verfahrens gemäß der vorliegenden Erfindung haben sich allerdings GlasKohlenstoffelektroden (glassy carbon electrode) nicht nur als geeignet für die Bestimmung von Dialkyldisulfiden erwiesen, sondern vielmehr als der einzige Arbeitselektrodentyp, der eine verlässliche Bestimmung von Dialkyldisulfiden erlaubt. Dies ist durchaus überraschend, denn nach gängiger Meinung in der Literatur zeigen Kohlenstoffelektroden, wie GlasKohlenstoffelektroden bei der Bestimmung von Disulfiden und Thiolen überhaupt keine Signale für Disulfide, sondern nur für Thiole (siehe C. Terashima et al., Analytical Chemistry, Vol. 75 No. 7, April 1, 2003, 1564 - 1572). Glaskohlenstoff-Elektroden bestehen typischerweise aus Kohlenstoff in Form von Stecknadeln oder Stäbchen mit einem Durchmesser von 2 bis 8 mm, die in einen Glas- oder Plastikhalter einzementiert oder eingepresst werden. Im Vergleich zu anderen Kohlenstoff-Elektroden wie Kohlepaste-Elektroden weisen Glaskohlenstoff-Elektroden zudem eine bessere chemische Widerstandsfähigkeit auf, da sie in Lösungsmitteln, wie Methanol oder Acetonitril, beständig sind. Falls erforderlich, können die Glaskohlenstoff-Elektroden durch Polieren mit einer Diamandpaste, gefolgt von einem Bad in einem Ultraschallbad mit hochreinem Wasser, auf ihre Verwendung vorbereitet werden.

Ein weiterer Vorteil von Glaskohlenstoffelektroden besteht darin, dass sie Messungen über einen weiten Bereich von ca. -0,8 V bis ca. 1,3 V erlauben. Im Rahmen der vorliegenden Erfindung hat sich gezeigt, dass in diesem weiten Potentialbereich die elektroaktiven schwefelhaltigen Ausgangsverbindungen, insbesondere Dialkyldisulfide und/oder Dialkylpolsulfide, vollständig oder zumindest nahezu vollständig zu nicht-elektroaktiven Spezies oxidiert werden. Mit Hilfe von Glaskohlenstoffelektroden kann daher die gepulste amperometrische Detektion im oxidativen Modus durchgeführt werden.

Hinsichtlich der Referenzelektrode unterliegt das Verfahren gemäß der vorliegenden Erfindung keinen Einschränkungen. Denkbar ist daher die Kombination einer Glaskohlenstoff-Elektrode als Arbeitselektrode mit sämtlichen zur Verfügung stehenden Elektroden erster Art oder zweiter Art als Referenzelektrode. Unter Elektroden erster Art werden im Rahmen der vorliegenden Erfindung und entsprechend dem allgemeinen Fachwissen des Fachmanns jegliche Elektroden verstanden, deren Potential direkt von der Konzentration der sie umgebenden Elektrolytlösung abhängt. Dies sind Feststoff-Elektroden wie zum Beispiel die Palladium-Elektrode. Im Rahmen der vorliegenden Erfindung und entsprechend dem allgemeinen Fachwissen des Fachmanns werden unter Elektroden zweiter Art diejenigen Elektroden verstanden, deren Potential nur indirekt von der sie umgebenden Elektrolytlösung abhängt. Durch die besondere Zusammensetzung der Elektrolytlösung wird das Potential der Elektrode konstant gehalten. Die Elektrolytlösung besteht zum einen aus einer gesättigten Lösung eines schwerlöslichen Salzes, dessen Kation aus dem gleichen Metall wie die Elektrode besteht und zum anderen aus einem gut löslichen Alkalisalz einer spezifischen Konzentration, welches das gleiche Anion wie das schwerlösliche Salz enthält. Das Potential hängt von der Konzentration des Kations des schwerlöslichen Salzes ab. Die Konzentration des Kations ist wiederum über das Löslichkeitsprodukt des Salzes mit der Konzentration des Anions gekoppelt. Wird die Konzentration des Anions konstant gehalten, bleibt daher auch das Potential konstant. Die Anionenkonzentration lässt sich dadurch nahezu konstant halten, indem ihr Wert sehr groß gewählt wird. Das tatsächliche Potential ergibt sich durch Subtraktion der Spannungswerte vom Messwert. Wichtige Referenzelektroden zweiter Art sind die Silber-Silberchlorid-Elektrode und die Kalomel-Elektrode. Auf Grund ihrer Zuverlässigkeit und unproblematischen Verwendung haben sich die Silber-Silberchlorid-Referenzelektrode und Palladiumreferenzelektrode als besonders vorteilhaft für das Verfahren gemäß der vorliegenden Erfindung erwiesen.

Vorzugsweise wird daher in der gepulsten amperometrischen Detektion eine Silber-Silberchlorid-Elektrode oder Palladium-Elektrode als Referenzelektrode eingesetzt.

Prinzipiell sind drei Varianten für die Durchführung einer gepulsten amperometrischen Detektion denkbar.

Bei der ersten Variante für die Durchführung einer gepulsten amperometrischen Detektion wird das Elektrodenpotential auf einen Bereich gepulst, in dem der Analyt elektroaktiv ist. Der zeitliche Verlauf des angelegten Potentials ähnelt daher einer Sprung- oder Heaviside-Funktion, wobei der Graph für den Potentialverlauf jedoch stetig verläuft und daher im Gegensatz zur Sprungfunktion keine Unterbrechung aufweist. Der zeitliche Verlauf des Messsignals zeichnet sich durch einen sprunghaften Anstieg auf einen Maximalwert für den Elektrolysestrom und eine sich unmittelbar daran anschließende Abnahme des gemessenen Stromes aus, was auf die Bildung einer Diffusionsschicht um die Elektrode und das fortdauernde Wachstum dieser Diffusionsschicht zurückgeführt wird.

Bei der zweiten Variante für die Durchführung einer gepulsten amperometrischen Detektion wird das Elektrodenpotential kurzfristig auf einen Bereich gepulst, in dem der Analyt elektroaktiv ist. In diesem Fall zeichnet sich der zeitliche Verlauf des an die Arbeitselektrode angelegten Potentials dadurch aus, dass das Anfangspotential in periodischen Zeitabständen für dieselbe Dauer durch jeweils identische rechteckige Potentialblöcke überlagert wird. Zwischen den Pulsen kann die sich an der Arbeitselektrode ausbildende Diffusionsschicht durch erzwungene oder natürliche Konvektion beseitigt werden. Der zeitliche Verlauf des gemessenen Elektrolysestroms zeichnet sich durch einen sprunghaftes Ansteigen und Abnehmen des Elektrolysestromes aus, was zeitlich einhergeht mit den jeweiligen Pulsen. Auf Grund der zeitlichen Begrenzung des höheren Elektrodenpotentials ist die Abnahme des gemessenen Elektrolysestroms auf die Dauer des Pulsens beschränkt und fällt daher entsprechend niedriger aus als bei der ersten Variante.

Die dritte Variante für die Durchführung einer gepulsten amperometrischen Detektion umfasst insgesamt drei Potentialprofile: ein erstes Potentialprofil zur Konditionierung der Elektrode, ein zweites zur Sorption des Analyten und schließlich ein drittes Potentialprofil zur Elektrooxidation des betreffenden Analyten. Der Elektrolysestrom wird nur bei Anliegen des dritten Potentialprofils gemessen.

Im Rahmen der vorliegenden Erfindung hat sich gezeigt, dass eine gepulste amperometrische Detektion mit mindestens drei unterschiedlichen Potentialprofilen während eines vollständigen Messzyklus eine besonders gute Reproduzierbarkeit und Verlässlichkeit der Messung erlaubt.

Vorzugsweise umfasst die gepulste amperometrische Detektion daher mindestens drei Potentialprofile.

Im Rahmen der vorliegenden Erfindung dient das niedrigste Potentialprofil (bzw. das elektronegativste Potentialprofil) zur Sorption des Analyten an der Arbeitselektrode und somit zur Konditionierung der Arbeitselektrode. Das höchste Potentialprofil (bzw. das elektropositivste Potentialprofil) dient im Rahmen der vorliegenden Erfindung zur vollständigen Oxidation von Adsorbaten an der Arbeitselektrode und bewirkt somit eine Reinigung der Arbeitselektrode. Ein weiteres Potentialprofil, dessen Höhe zwischen dem niedrigsten und dem höchsten Potentialprofil liegt, dient im Rahmen der vorliegenden Erfindung zur Elektrooxidation des zu bestimmenden Dialkyldisulfids; es dient somit auch zur Messung des betreffenden Elektrolysestroms und zur Bestimmung des betreffenden Dialkyldisulfids.

Vorzugsweise bildet im Rahmen der vorliegenden Erfindung die Abfolge aus Elektrooxidation bei einem Oxidationspotential, vollständiger Oxidation bei einem Reinigungspotential und Sorption des Analyten bei einem Konditionierungspotential einen Puls für eine gepulste amperometrische Detektion. Vorteilhafterweise schließt sich einem Oxidationspotential ein Reinigungspotential an. Dies hat den Vorteil, dass die während der Elektrooxidation nicht vollständig zu nicht-elektroaktiven Spezies oxidierten Verbindungen oder gegebenenfalls Verunreinigungen bzw. Rückstände an der Arbeitselektrode durch das Anlegen eines gegenüber dem Oxidationspotential elektropositiveren Reinigungspotentials vollständig von der Arbeitselektrode entfernt werden und die nachfolgende(n) Messung(en) daher nicht beeinträchtigen. Vorzugsweise schließt sich dem Reinigungspotential ein Konditionierungspotential an. Das Anlegen eines Konditionierungspotentials erleichtert die Sorption des zu bestimmenden Analyten an der Elektrodenoberfläche. Dies wiederum erhöht die Messgenauigkeit der anschließenden Messung des Elektrolysestroms während der Zeitdauer, in der ein Oxidationspotential an der Arbeitselektrode anliegt. Hinsichtlich der Anzahl einzelner spezifischer Potentialprofile, die in einer gepulsten amperometrischen Detektion eingesetzt werden können, unterliegt das erfindungsgemäße Verfahren keinerlei Beschränkung.

Vorzugsweise umfasst die gepulste amperometrische Detektion daher mindestens ein Oxidationspotential, mindestens ein Reinigungspotential und mindestens ein Konditionierungspotential.

In Abhängigkeit von dem zu bestimmenden Dialkyldisulfid können die spezifischen Werte für die individuellen Potentialprofile so eingestellt werden, dass während der Zeit, in der die jeweiligen Potentialprofile Oxidationspotential, Reinigungspotential und Konditionierungspotential an der Arbeitselektrode anliegen, nur die dem einzelnen Potentialprofil zugewiesenen Effekte eintreten.

Im Rahmen der vorliegenden Erfindung hat sich gezeigt, dass eine Elektrooxidation bei einem Oxidationspotential im Bereich von ca. + 0,5 V bis ca. + 1,3 V sich für die Bestimmung sämtlicher gängigen Dialkyldisulfide eignet und insbesondere für die Bestimmung von Dimethyldisulfid, welches eine Zwischenstufe bei der Herstellung der kommerziell bedeutenden Methansulfonsäure ist. Ferner hat sich gezeigt, dass ein Reinigungspotential im Bereich von mindestens ca. + 1,5 V ausreichend ist, um eine vollständige Reinigung der Arbeitselektrode von Adsorbaten bzw. verbliebenen Verschmutzungen zu gewährleisten. Zur Konditionierung der Arbeitselektrode hat sich außerdem ein Konditionierungspotential im Bereich von ca. - 0,5 V bis ca. + 0,5 V als geeignet erwiesen, um die Sorption des Analyten für die sich anschließende Elektrooxidation zu erleichtern. Im Rahmen der vorliegenden Erfindung bezeichnet der Ausdruck ca. im Zusammenhang mit der Angabe von Potentialwerten eine Abweichung von +/- 10 % von dem explizit genannten Wert.

Vorzugsweise hat daher das Oxidationspotential bei der gepulsten amperometrischen Detektion einer schwefelhaltigen Ausgangsverbindung in Schritt b) des erfindungsgemäßen Verfahrens einen Wert von ca. + 0,5 V bis ca. + 1,3 V, das Reinigungspotential einen Wert von mindestens ca. + 1,3 V und das Konditionierungspotential einen Wert von ca. - 0,5 V bis ca. + 0,5 V.

Für die Bestimmung von Dialkyldisulfiden in Alkansulfonsäuren, insbesondere für die Bestimmung von Dimethyldisulfid in Methansulfonsäure, haben sich ein Oxidationspotential mit einem Wert von ca. + 0, 8 V bis ca. 1,2 V, ein Reinigungspotential mit einem Wert von mindestens ca. + 1,5 V und ein Konditionierungspotential mit einem Wert von ca. - 0, 3 V bis ca. + 0,3 V als besonders geeignet erwiesen, um präzise und reproduzierbare Ergebnisse zu gewährleisten.

Insbesondere hat deshalb das Oxidationspotential bei der gepulsten amperometrischen Detektion einer schwefelhaltigen Ausgangsverbindung in Schritt b) des erfindungsgemäßen Verfahrens einen Wert von ca. + 0,8 V bis ca. 1,2 V, das Reinigungspotential einen Wert von mindestens ca. + 1,5 V und das Konditionierungspotential einen Wert von ca. - 0,3 V bis ca. + 0,3 V.

Das Anlegen dieser und vorangegangener Potentialprofile an die Arbeitselektrode unterliegt keinerlei Beschränkungen. Beispielsweise kann ein spezifisches Potential mit einem konstanten Wert aus den vorstehend genannten Bereichen für das betreffende Potential während der gesamten Zeitdauer in der das betreffende Elektrodenpotential an die Arbeitselektrode angelegt wird. Denkbar ist auch ein Rampenverlauf für das betreffende Potential: In diesem Fall liegt der Wert des betreffenden Potentials an der Arbeitselektrode zu Beginn der Zeitdauer deutlich unter seinem Maximalwert, im Grenzfall handelt es sich um seinen Minimalwert, und bis zum Ende der Zeitdauer wird sein Wert mit einem konstanten Potentialanstieg pro definierter Zeiteinheit erhöht, im Grenzfall auf seinen Maximalwert.

Damit gewährleistet wird, dass die durch Anlegen der jeweiligen Potentialprofile an die Arbeitselektrode erstrebten Zwecke auch tatsächlich eintreten, müssen die betreffenden Potentiale für eine ausreichende Zeit an der Arbeitselektrode anliegen. Unabhängig von dem zu bestimmenden Dialkyldisulfid haben sich die folgenden Zeiträume als günstig erwiesen, damit die den Potentialprofilen Oxidationspotential, Reinigungspotential und Konditionierungspotential zugewiesenen Effekte auch eintreten können: mindestens ca. 60 ms für das Oxidationspotential, mindestens ca. 10 ms für das Reinigungspotential und mindestens ca. 40 ms für das Konditionierungspotential. Im Rahmen der vorliegenden Erfindung bezeichnet der Ausdruck ca. im Zusammenhang mit der Angabe von Zeitintervallen eine Abweichung von +/- 10 % von dem explizit genannten Wert. Abweichungen in dieser Größenordnung führen allgemein nicht zu einer merklichen Verschlechterung des Signal-Rausch-Verhältnisses.

Vorzugsweise beträgt daher die Dauer des Oxidationspotentials bei der gepulsten amperometrischen Detektion einer schwefelhaltigen Ausgangsverbindung in Schritt b) des erfindungsgemäßen Verfahrens mindestens ca. 60 ms, die Dauer des Reinigungspotentials mindestens ca. 10 ms und die Dauer des Konditionierungspotentials mindestens ca. 40 ms.

Auch wenn in dem Zeitraum, in dem das Oxidationspotential an der Arbeitselektrode anliegt, prinzipiell der entsprechende Elektrolysestrom für das betreffende Dialkyldisulfid fließt, wird dennoch nicht während des ganzen Zeitraums der Elektrolysestrom gemessen. Denn zu Beginn der Dauer des Oxidationspotentials ist der Elektrolysestrom noch nicht konstant. Um aus den Schwankungen des Elektrolysestrom resultierende Messungenauigkeiten zu vermeiden, wird daher der Elektrolysestrom erst gemessen, nachdem sich dieser auf einen konstanten Wert eingestellt hat. Typischerweise wird der Elektrolysestrom erst in der letzten Hälfte der Dauer des Oxidationspotentials, bevorzugt erst im letzten Drittel der Dauer des Oxidationspotentials gemessen.

Der Elektrolysestrom als solcher stellt ein Kurzzeitsignal dar, für dessen möglichst genaue Messung das Signal-Rausch-Verhältnis relevant ist. Für die Messungen von kurzzeitigen amperometrischen Signalen wird das Signal-Rausch-Verhältnis durch die instrumentelle Vorgehensweise beeinflusst, die zum Abfragen des Elektrodenstroms verwendet wird. Eine wesentliche Rauschkomponente der amperometrischen Bestimmung bei konstantem Elektrodenpotential, also ohne Pulsen des Arbeitselektrodenpotentials, ist sinusförmig und korreliert mit der 60 Hz-Zeilenwechselfrequenz. Um möglichst genaue Messungen mit Hilfe der gepulsten amperometrischen Detektion zu erzielen, wird daher im Rahmen der vorliegenden Erfindung ein Signal verwendet, dass durchschnittlich einem Mehrfachen der Periode einer einzelnen 60 Hz-Oszillation entspricht, also einer Schwingungsdauer von 16,7 ms. In diesem Fall gibt es daher keinen Beitrag des sinusförmigen 60 Hz-Rauschsignals zur Signalstärke. Das Zeit-Integral eines sinusförmigen 60 Hz-Rauschsignals hat den Wert 0 für jedes beliebige ganzzahlige Vielfache $v$ der Perioden mit einer Schwingungsdauer von 16,7 ms. Die analytische Signalstärke kann daher für Vielfache $v$ der Schwingungsdauer, die deutlich größer als 1 sind, beträchtlich gesteigert werden. Wenn also das analytische Signal während der gesamten Periode von $v* 16,7$ ms einen konstanten Wert hat, dann wird das Signal-Rausch-Verhältnis um den Faktor $v$ verstärkt. Im Rahmen der vorliegenden Erfindung haben sich die ganzzahligen Vielfachen $v$ der Schwingungsdauer von 16,7 ms von mindestens 6 als günstig erwiesen, um präzise und reproduzierbare Ergebnisse für den in der letzten Hälfte, bevorzugt im letzten Drittel der Dauer des Oxidationspotentials gemessenen Elektrolysestrom zu gewährleisten.

Vorzugsweise beträgt daher die Messdauer bei der gepulsten amperometrischen Detektion einer schwefelhaltigen Ausgangsverbindung in Schritt b) des erfindungsgemäßen Verfahrens ein ganzzahliges Vielfaches von 16,7 ms.

Bei einer Dauer von mindestens 300 ms für das Oxidationspotential liegt die Messdauer für den Elektrolysestrom zwischen ca. 100 ms und 150 ms. Bevorzugt beträgt die Messdauer dann ca. 50 ms, ca. 67 ms, ca. 84 ms, ca. 100 ms, ca. 117 ms, ca. 134 ms und ca. 150 ms. In diesen Fällen handelt sich um das 3-fache, 4-fache, 5-fache, 6-fache, 7-fache, 8-fache oder 9-fache der Schwingungsdauer von 16,7 ms.

Bevorzugt beträgt daher die Dauer des Oxidationspotentials bei der gepulsten amperometrischen Detektion einer schwefelhaltigen Ausgangsverbindung in Schritt b) des erfindungsgemäßen Verfahrens mindestens ca. 300 ms.

Bei einer Dauer des Oxidationspotentials von mindestens ca. 300 ms beträgt die Dauer des Reinigungspotentials vorzugsweise mindestens ca. 50 ms und die Dauer des Konditionierungspotentials vorzugsweise mindestens ca. 200 ms.

Eine typische Dauer für einen sich aus Oxidationspotential, Reinigungspotential und Konditionierungspotential zusammensetzenden Messzyklus hat somit eine Gesamtdauer von mindestens ca. 500 ms.

Typischerweise wird der Elektrolysestrom erst in der letzten Hälfte der Dauer des Oxidationspotentials, bevorzugt erst im letzten Drittel der Dauer des Oxidationspotentials gemessen. Daher beträgt die Dauer seiner Messung vorzugsweise ein ganzzahliges Vielfaches von 16,7 ms und liegt insbesondere zwischen der Hälfte und einem Drittel der Dauer des Oxidationspotentials.

Um ausreichende Kenntnis über den Fortschritt der Oxidation der schwefelhaltigen Ausgangsverbindungen zur korrespondierenden Alkansulfonsäure und die gegebenenfalls erforderliche Menge zusätzlichen Oxidationsmittels zur Vervollständigung der Oxidationsreaktion zu haben, ist es auch erforderlich, die schwefelhaltige Ausgangsverbindung im Reaktionsaustrag von Schritt a) zu bestimmen. Typischerweise wird ein Dialkyldisulfid und/oder ein Dialkylpolysulfid als schwefelhaltige Ausgangsverbindung zur Herstellung von Alkansulfonsäuren eingesetzt. Hergestellt werden die Dialkyldisulfide dann in einer dem Schritt a) vorgelagerten Reaktion, beispielsweise in einer basenkatalysierten Oxidation von Alkylmercaptanen mit Schwefel unter Freisetzung von Schwefelwasserstoff, gefolgt von einer Aufreinigung der Rohdialkyldisulfide, bevorzugt durch eine Destillation. Dies hat den Vorteil, dass eine zumindest im Wesentlichen reine schwefelhaltige Ausgangsverbindung der Oxidation von Schritt a) zugeführt wird. Auf diese Weise wird die Bildung möglicher Nebenprodukte vermieden und gleichzeitig bleibt die Menge der gebildeten Oxidationszwischenprodukte überschaubar.

In einer weiteren Ausführungsform des erfindungsgemäßen Verfahrens wird daher in Schritt b) ein Dialkyldisulfid und/oder ein Dialkylpolysulfid als schwefelhaltige Ausgangsverbindung bestimmt.

Wie bereits erläutert können Dialkyldisulfide bzw. Dialkylpolysulfide mit Hilfe der Amperometrie bestimmt werden, da sie über freie Elektronenpaare an Schwefelatomen verfügen, auf Grund derer sie an Elektrodenoberflächen absorbieren können, um dort elektrochemisch oxidiert zu werden. Allerdings verfügen auch manche der bei der Oxidation von Dialkyldisulfiden bzw. Dialkylpolysulfiden auftretenden Oxidationszwischenprodukte, insbesondere Alkansulfonsäure-S-alkylester, über freie Elektronenpaare an zumindest einem der Schwefelatome. Folglich können diese Oxidationszwischenprodukte auch an der Arbeitselektrode sorbiert und dort elektrochemisch oxidiert werden. Dies würde jedoch die Messung des Elektrolysestroms bei der gepulsten amperometrischen Bestimmung der schwefelhaltigen Ausgangsverbindung beeinflussen und zu höheren Werte für das zu bestimmende Dialkyldisulfid bzw. Dialkylpolysulfid führen. Dies ist insbesondere dann problematisch, wenn die UV-Vis-Bestimmung von Oxidationszwischenprodukten, insbesondere von Alkansulfonsäure-S-alkylestern, der gepulsten amperometrischen Bestimmung von Dialkyldisulfiden bzw. Dialkylpolysulfiden nachgeschaltet ist. Denn dann würden keine Alkansulfonsäure-S-alkylester im Reaktionsaustrag aus Schritt a) bestimmt werden, auch wenn sie in diesem tatsächlich vorhanden waren. Als Folge dessen könnten die Alkansulfonsäure-S-alkylester im ungünstigsten Fall in die der Produktion nachgeschalteten Destillationsapparaturen gelangen und dort zu Niederschlägen von elementarem Schwefel und somit zu Betriebsstörungen führen.

Die Leistungsfähigkeit des erfindungsgemäßen Verfahrens kann dadurch signifikant erhöht werden, indem zunächst eine Probe aus dem Reaktionsaustrag aus Schritt a) entnommen und diese Probe in zwei separate Proben aufgeteilt wird. Anschließend wird die Bestimmung von Oxidationszwischenprodukten, insbesondere Alkansulfonsäure-S-alkylestern, bevorzugt mittels UV-Vis-Spektroskopie aus der einen separaten Probe und die Bestimmung von schwefelhaltigen Ausgangsverbindungen, insbesondere Dialkyldisulfiden und/oder Dialkylpolysulfiden, bevorzugt mittels amperometrischer Detektion aus der anderen separaten Probe vorgenommen.

In einer Ausführungsform des erfindungsgemäßen Verfahrens erfolgen daher die Bestimmung von Oxidationszwischenprodukten und die Bestimmung von schwefelhaltigen Ausgangsverbindungen unabhängig voneinander in separaten Proben aus dem Reaktionsaustrag aus Schritt a).

Alternativ können sowohl die Genauigkeit als auch die Verlässlichkeit bei der Bestimmung von schwefelhaltigen Ausgangsverbindungen, insbesondere von Dialkyldisulfiden und Dialkylpolysulfiden, in Anwesenheit von Oxidationszwischenprodukten aus Schritt a) dadurch verbessert werden, indem vor der Bestimmung in Schritt b) die Probe aus dem Reaktionsaustrag aus Schritt a) einer chromatographischen Trennung unterworfen wird. Die schwefelhaltigen Ausgangsverbindungen, insbesondere Dialkyldisulfide und Dialkylpolysulfide, sind deutlich weniger polar als die Oxidationszwischenprodukte, insbesondere Alkansulfonsäure-S-alkylester. Daher unterscheiden sich die schwefelhaltigen Ausgangsverbindungen und Oxidationszwischenprodukte in ihrem jeweiligen Retentionsverhalten bei einer chromatographischen Trennung. Aus diesem Grund werden schwefelhaltige Ausgangsverbindungen nach anderen Retentionszeiten als die Oxidationszwischenprodukte von der Trennsäule eluiert. Dies hat den Vorteil, dass die zu bestimmenden Verbindungen zu unterschiedlichen Zeitpunkten in die Messzelle für die gepulste amperometrische Detektion eintreten und sich somit bei den individuellen Bestimmungen nicht gegenseitig stören, was insbesondere zu einer Verbesserung der Genauigkeit und Verlässlichkeit in der Bestimmung von Dialkyldisulfiden und/oder Dialkylpolysulfiden mit Hilfe der gepulsten amperometrischen Detektion führt.

Ferner erlaubt eine chromatographische Trennung einer Probe aus dem Reaktionsaustrag aus Schritt a) des erfindungsgemäßen Verfahren, die der Bestimmung schwefelhaltiger Ausgangsverbindungen und/oder von Oxidationszwischenprodukten in Schritt b) des erfindungsgemäßen Verfahrens vorangeht, auch die Erstellung von separaten Proben, die jeweils unterschiedliche Verbindungen aus der Probe aus dem Reaktionsaustrag aus Schritt a) enthalten. Vorzugsweise enthält eine erste Probe nur eine schwefelhaltige Ausgangsverbindung, beispielsweise ein Dialkyldisulfid und/oder Dialkylpolysulfid enthält, und eine andere Probe enthält nur ein Oxidationszwischenprodukt, beispielsweise einen Alkansulfonsäure-S-alkylester.

In einer Ausführungsform umfasst das erfindungsgemäße Verfahren daher zusätzlich den dem Schritt b) vorgelagerten Schritt der chromatographischen Trennung einer Probe aus dem Reaktionsaustrag aus Schritt a).

Im Rahmen der vorliegenden Erfindung hat sich die Umkehrphasenchromatographie (reversed phase chromatography) als eine geeignete chromatographische Methode zur Trennung von schwefelhaltigen Ausgangsverbindungen, insbesondere Dialkyldisulfiden und/oder Dialkylpolysulfiden, und Oxidationszwischenprodukten, insbesondere Alkansulfonsäure-S-alkylestern, erwiesen. Bei Verwendung einer Umkehrphasenchromatographie (reversed phase chromatography) mit einem UV-Vis-Spektrometer wird beispielsweise Methansulfonsäure-S-methylester bereits nach 5,6 Minuten detektiert, während Dimethyldisulfid erst nach 36,6 Minuten detektiert wird. Die Differenz zwischen den Retentionszeiten von Dialkyldisulfiden bzw. Dialkylpolysulfiden und von Alkansulfonsäure-S-alkylestern ist also groß genug, um diese Verbindungen gut voneinander trennen zu können. Dies ermöglicht eine zuverlässige und präzise Bestimmung von Dialkyldisulfiden bzw. Dialkylpolysulfiden und Alkansulfonsäure-S-alkylestern.

Bevorzugt wird daher die chromatographische Trennung als Umkehrphasen-Chromatographie (reversed phase chromatography) durchgeführt.

Hinsichtlich der Ausgestaltung der Durchführung dieser Umkehrphasen-Chromatographie unterliegt das Verfahren gemäß der vorliegenden Erfindung prinzipiell keinen Einschränkungen. Daher sind grundsätzlich alle denkbaren Ausgestaltungen der Umkehrphasen-Chromatographie, wie Ionenchromatographie und Hochleistungsflüssigkeits-Chromatographie, im erfindungsgemäßen Verfahren einsetzbar. Im Rahmen der vorliegenden Erfindung hat sich jedoch die Ionenchromatographie als besonders geeignet erwiesen, da sie mit überschaubarem Aufwand eine vollständige Trennung der Probe aus dem Reaktionsaustrag aus Schritt a) des erfindungsgemäßen Verfahrens in seine einzelnen Komponenten erlaubt. Zudem hat die Ionenchromatographie gegenüber der Hochleistungschromatographie den Vorteil, dass sie eine größere Anzahl von Möglichkeiten zur Einstellung von Parametern für (möglichst) optimale Trennbedingungen erlaubt.

Besonders bevorzugt wird daher die chromatographische Trennung als Ionenchromatographie durchgeführt.

Polare Lösungsmittel, die sich zur Durchführung der Umkehrphasen-Chromatographie für schwefelhaltige Ausgangsverbindungen, insbesondere Dialkyldisulfide und/oder Dialkylpolysulfide, und Oxidationszwischenprodukte, insbesondere Alkansulfonsäure-S-alkylester, eignen, sind protische Lösungsmittel wie Methanol, Ethanol und Wasser oder Gemische protischer Lösungsmittel. Im Rahmen der vorliegenden Erfindung wird der Begriff protisches Lösungsmittel entsprechend dem allgemeinen Fachwissen des Fachmanns verwendet und bezeichnet daher solche Verbindungen, die über eine funktionelle Gruppe verfügen, aus der Wasserstoffatome im Molekül entweder ohne Einwirkung von Basen oder durch Einwirkung schwacher Basen als Protonen abgespalten werden können. Als besonders geeignet für die Durchführung der chromatographischen Trennung hat sich im Rahmen der vorliegenden Erfindung ein Gemisch aus Methanol und Wasser erwiesen.

Bevorzugt umfasst das erfindungsgemäße Verfahren daher zusätzlich den der Bestimmung in Schritt b) vorangehenden Schritt des Auflösens einer Probe aus dem Reaktionsaustrag aus Schritt a) in einem protischen Lösungsmittel oder einem Gemisch aus protischen Lösungsmitteln, insbesondere in einem Gemisch aus Methanol und Wasser.

Für eine möglichst vollständige Trennung hat sich im Rahmen der vorliegenden Erfindung eine mobile Phase auf Basis einer Mischung von 70 Vol.-% +/- 10 Vol.-% Wasser mit 30 Vol.-% +/- 10 Vol.-% Methanol als geeignet erwiesen.

Besonders bevorzugt umfasst das erfindungsgemäße Verfahren daher zusätzlich den der Bestimmung in Schritt b) vorangehenden Schritts des Auflösens einer Probe aus dem Reaktionsaustrag aus Schritt a) in einem Gemisch aus Methanol und Wasser in einem Volumenverhältnis von Methanol zu Wasser von 2:8 bis 4:8.

Bevorzugt wird durch das Auflösen einer Probe aus dem Reaktionsaustrag aus Schritt a) in einem protischen Lösungsmittel oder einem Gemisch aus protischen Lösungsmitteln die Probe auch um einen Faktor von 10 bis 1000 verdünnt.

Der für die chromatographische Trennung verwendete Eluent durchfließt nach Austritt aus der Chromatographiesäule mit der Probe die amperometrische Messzelle. Daher muss das zur Lösung der Probe verwendete protische Lösungsmittel oder das Gemisch aus protischen Lösungsmitteln nicht nur die Funktion eines Eluenten in der chromatographischen Trennung erfüllen, sondern auch die eines Elektrolyten in der anschließenden Bestimmung des Dialkyldisulfids mittels gepulster amperometrischer Detektion. Dies setzt eine Eigenleitfähigkeit des betreffenden Lösungsmittels oder Lösungsmittelgemisches voraus, die im Regelfall nicht allein durch zusätzlich gegebenenfalls in der Probe enthaltene Ionen wie Chlorid, Nitrat oder dergleichen bewirkt wird. Im einfachsten Fall wird die erforderliche Leitfähigkeit durch die in der Probe anwesende Alkansulfonsäure, vorzugsweise Methansulfonsäure, herbeigeführt.

Im Wesentlichen unterliegt das erfindungsgemäße Verfahren keinen Einschränkungen hinsichtlich des Oxidationsmittels, solange gewährleistet ist, dass das Oxidationsmittel dazu geeignet ist, eine schwefelhaltige Ausgangsverbindung und die aus der Oxidation der schwefelhaltigen Ausgangsverbindung resultierenden Oxidationszwischenprodukte zu den gewünschten Alkansulfonsäuren zu überführen. Ein geeignetes Oxidationsmittel im erfindungsgemäßen Verfahren ist Sauerstoff, und zwar sowohl in freier Form als auch in gebundener Form. Im Rahmen der vorliegenden Erfindung wird der Begriff Sauerstoff in freier Form entsprechend dem allgemeinen Fachwissen des Fachmanns verwendet und bezeichnet Sauerstoff, der nicht durch kovalente Bindung(en) Teil einer organischen oder anorganischen Verbindung ist. Sauerstoff in freier Form ist beispielsweise molekularer Sauerstoff $O_2$, Ozon $O_3$ oder ein Sauerstoffradikal. Gemäß diesem Verständnis ist daher beispielsweise ein Sauerstoffmolekül oder ein Sauerstoffradikal, das Teil eines Komplexes ist bzw. in koordinierter Form vorliegt, auch als Sauerstoff in freier Form zu verstehen. Als Sauerstoff in freier Form kann daher im erfindungsgemäßen Verfahren unter anderem reiner Sauerstoff oder ein mit Sauerstoff angereicherter Gasstrom, wie zum Beispiel mit Sauerstoff angereicherte Luft oder eine Mischung aus reinem Sauerstoff und einem unter den Oxidationsbedingungen nicht reaktivem sogenannten Inertgas, beispielsweise Stickstoff oder Argon, verwendet werden. Im Gegensatz dazu bezeichnet der Begriff Sauerstoff in gebundener Form jeglichen Sauerstoff, der durch mindestens eine kovalente Bindung Teil einer anorganischen oder organischen Verbindung ist. Im Ergebnis dienen die Verbindungen mit Sauerstoff in gebundener Form zur Übertragung von Sauerstoffatomen auf die im Dialkyldisulfid enthaltenen Schwefelatome, um diese von der formalen Oxidationsstufe -1 im Dialkyldisulfid (schrittweise) bis zur Oxidationsstufe +3 im Dialkyldisulfon zu oxidieren. Alternativ können im Rahmen der vorliegenden Erfindung auch gleichzeitig Sauerstoff in freier Form und Sauerstoff in gebundener Form zur Oxidation einer schwefelhaltigen Ausgangsverbindung, insbesondere zur Oxidation des Dialkyldisulfids und/oder Dialkylpolysulfids eingesetzt werden.

In einer Ausführungsform des erfindungsgemäßen Verfahrens wird daher der Schritt a) mit freiem Sauerstoff und/oder mit Sauerstoff in gebundener Form durchgeführt.

Bevorzugt wird das erfindungsgemäße Verfahren in Anwesenheit katalytischer Mengen Salpetersäure durchgeführt. Der Begriff katalytische Mengen Salpetersäure bezeichnet im Rahmen der vorliegenden Erfindung sämtliche Mengen Salpetersäure, die kleiner sind als die zur Oxidation einer erfindungsgemäß eingesetzten schwefelhaltigen Verbindung erforderliche stöchiometrische Menge Salpetersäure. Insbesondere bezeichnet der Ausdruck katalytische Mengen Salpetersäure ein Verhältnis von schwefelhaltiger Ausgangsverbindung, bevorzugt Dialkyldisulfid, zu Salpetersäure von 2000:1 (mol/mol) bis 1:1 (mol/mol), wobei dieses Verhältnis sämtliche durch ganze und reelle Zahlen ausdrückbaren Werte von einschließlich 2000:1 (mol/mol) bis einschließlich 1:1 (mol/mol) beinhaltet. Dieses Verhältnis umfasst insbesondere die Werte 2000:1 (mol/mol), 1000:1 (mol/mol), 500:1 (mol/mol), 200:1 (mol/mol), 100:1 (mol/mol), 90:1 (mol/mol), 80:1 (mol/mol), 70:1 (mol/mol), 60:1 (mol/mol), 50:1 (mol/mol), 40:1 (mol/mol), 30:1 (mol/mol), 20:1 (mol/mol), 10:1 (mol/mol), 5:1 (mol/mol), 2:1 (mol/mol) und 1:1 (mol/mol). Im Rahmen der vorliegenden Erfindung beziehen sich diese Werte auf die Konzentrationen der betreffenden Komponenten in der Reaktionsmischung zu Beginn der Reaktion.

Vorzugsweise wird im erfindungsgemäßen Verfahren zur Oxidation Luft, ein mit Sauerstoff in freier Form angereicherter Gasstrom und/oder reiner Sauerstoff in freier Form eingespeist, wobei die Verwendung von Sauerstoff in freier Form oder von einem mit Sauerstoff in freier Form angereicherten Gasstrom zur Oxidation besonders bevorzugt ist. Denn durch den molekularen Sauerstoff sowie das im Reaktionsgemisch anwesende Wasser werden die Stickstoffoxide, die sowohl durch die Oxidationsreaktion als auch durch thermische Zersetzung gebildet haben, wieder zur Salpetersäure regeneriert. Bei der Durchführung des erfindungsgemäßen Verfahrens mit Salpetersäure als Oxidationsmittel bzw. als Katalysator findet diese Regenerierung automatisch immer als Nebenreaktion statt. Dies hat den Vorteil, dass eine kontinuierliche Zugabe von Salpetersäure im erfindungsgemäßen Verfahren vorzugsweise nicht erforderlich ist.

Falls jedoch die Stickstoffoxide nicht vollständig zur Salpetersäure regeneriert werden, und es dadurch zu einem Verlust an Salpetersäure kommen sollte, kann dieser Verlust durch Zugabe von frischer Salpetersäure kompensiert werden. Diese Supplementierung von nicht regenerierter und somit verloren gegangener Salpetersäure kann in Abhängigkeit von den jeweils erforderlichen Mengen an frischer Salpetersäure entweder sporadisch oder kontinuierlich erfolgen.

Die Oxidation schwefelhaltiger Ausgangsverbindungen, insbesondere des Dialkyldisulfids und/oder Dialkylpolysulfids mit Hilfe eines Gasstromes, der mit Sauerstoff in freier Form angereichert ist, hat den Vorteil, dass ein gegenüber reinem Sauerstoff relativ günstiger Gasstrom in die Reaktion eingespeist wird. Zudem kann entsprechend dem Verlauf der Oxidationsreaktion der Gehalt an Sauerstoff im einzuspeisenden Gasstrom nach Belieben eingestellt werden. Im einfachsten Fall handelt es sich bei diesem Gasstrom um Luft mit mehr als 20,9 Vol.-% (Volumenprozent) Sauerstoff in freier Form.

Durch Einspeisung eines Gasstromes mit mindestens 21 Vol.-% Sauerstoff in freier Form in die Reaktionsmischung wird gewährleistet, dass sowohl eine möglichst vollständige Oxidation des Dialkyldisulfids zur entsprechenden Alkansulfonsäure als auch eine Regenerierung der Stickstoffoxide $NO_x$ zur Salpetersäure erreicht wird.

Vorzugsweise wird daher zur Oxidation ein Sauerstoff in freier Form enthaltender Gasstrom mit einem Gehalt von mindestens 21 Vol.-% Sauerstoff in freier Form, insbesondere mehr als 21 Vol.-% Sauerstoff in freier Form, eingespeist.

Im Rahmen der vorliegenden Erfindung bezeichnet der Ausdruck mindestens 21 Vol.-% Sauerstoff in freier Form alle Werte von größer oder gleich 21 Vol.-% bis einschließlich 100 Vol.-%, die sich durch ganze und durch reelle Zahlen ausdrücken lassen. Im Grenzfall handelt es sich bei dem Gasstrom mit einem Gehalt von mindestens 21 Vol.-% Sauerstoff in freier Form um reinen Sauerstoff in freier Form, vorzugsweise um molekularen Sauerstoff $O_2$.

Hinsichtlich der Anzahl von Reaktoren in denen die Oxidation gemäß Schritt a) des erfindungsgemäßen Verfahrens durchgeführt wird, unterliegt das erfindungsgemäße Verfahren prinzipiell keinen Einschränkungen. Beim Einsatz von zwei oder mehr Reaktoren im erfindungsgemäßen Verfahren können die Reaktoren sowohl parallel als auch seriell angeordnet sein. Denkbar ist auch eine Mischung von parallel und seriell angeordneten Reaktoren.

In einer Ausführungsform des erfindungsgemäßen Verfahrens wird daher der Schritt a) in mindestens einem Reaktor durchgeführt.

Findet Schritt a) des erfindungsgemäßen Verfahrens nur in einem einzigen Reaktor statt, wird in Schritt c) die Menge zusätzlichen Oxidationsmittels zur Überführung der in Schritt b) bestimmten schwefelhaltigen Ausgangsverbindungen und/oder Oxidationszwischenprodukte auch in diesen Reaktor eingespeist. Diese Verfahrensführung kann jedoch nicht verhindern, dass die Oxidation der schwefelhaltigen Ausgangsverbindungen zur gewünschten Alkansulfonsäure nicht immer vollständig verläuft, selbst wenn das zur Oxidation erforderliche Oxidationsmittel in einer stöchiometrisch ausreichenden Menge in den Reaktor eingespeist wird. Variiert beispielsweise die Konzentration der schwefelhaltigen Ausgangsverbindung im Zuführstrom, kann es vorkommen, dass die in den Reaktor eingespeiste Menge des Oxidationsmittels nicht ausreicht, um eine vollständige Oxidation der Ausgangsverbindungen zur Alkansulfonsäure zu gewährleisten. Ferner kann die Verweilzeit der Reaktanden im Reaktor zu kurz oder die Ausgangsverbindungen und das Oxidationsmittel nicht ausreichend durchmischt sein, um eine vollständige Oxidation der Ausgangsverbindungen zu erzielen.

Vorzugsweise wird der Schritt a) des erfindungsgemäßen Verfahrens daher in zwei seriell angeordneten Reaktoren durchgeführt, wobei der Reaktionsaustrag aus der im ersten Reaktor durchgeführten Oxidation in den zweiten Reaktor und gegebenenfalls in weitere Reaktoren eingeleitet wird. Abhängig vom Grad des Umsatzes der schwefelhaltigen Ausgangsverbindungen für die die im ersten Reaktor durchgeführte Oxidationsreaktion kann der Schritt a) des erfindungsgemäßen Verfahrens in sämtlichen Reaktoren einer Serie von Reaktoren stattfinden. Dies hat den Vorteil, dass eine im Wesentlichen vollständige Umsetzung der schwefelhaltigen Ausgangsverbindungen zur gewünschten Alkansulfonsäuren erreicht wird. Dadurch wird vermieden, dass sich in der Reaktionsmischung nicht umgesetzte schwefelhaltige Ausgangsverbindungen und/oder Oxidationsstufen akkumulieren.

In einer bevorzugten Ausführungsform des erfindungsgemäßen Verfahrens wird daher der Schritt a) in mindestens zwei seriell angeordneten Reaktoren durchgeführt.

Im Rahmen des erfindungsgemäßen Verfahrens umfasst die Durchführung des Schritts a) in mindestens zwei seriell angeordneten Reaktoren sowohl eine Reihe seriell angeordneter Reaktoren als auch zwei oder mehrere Reihen seriell angeordneter Reaktoren, wobei die zwei oder mehre Reihen dann parallel zueinander angeordnet sind.

Bei Durchführung des erfindungsgemäßen Verfahrens in mindestens zwei seriell angeordneten Reaktoren, wird das erfindungsgemäße Verfahren in einer Kombination eines Hauptreaktors mit einem Nachreaktor durchgeführt. Der Nachreaktor dient zur Vervollständigung der Oxidationsreaktion und wird daher auch als Finisher-Reaktor bezeichnet. Beispielsweise kann ein kontinuierlich betriebener Rührkesselreaktor als Hauptreaktor, in welchem die größte Menge der schwefelhaltigen Ausgangsverbindungen, insbesondere des Dialkyldisulfids und/oder Dialkylpolysulfids, umgesetzt wird, mit einem Strömungsrohr als Nachreaktor zur Vervollständigung der Reaktion kombiniert werden.

Um sicherzustellen, dass das Oxidationsmittel in der zur Vervollständigung der Oxidationsreaktion erforderlichen Menge in den zweiten Reaktor und gegebenenfalls in weitere Reaktoren eingespeist wird, muss der Schritt b) des erfindungsgemäßen Verfahrens nach dem Reaktor erfolgen, der demjenigen Reaktor voranschreitet, dem der Reaktionsaustrag zugeführt und in den das zusätzliche Oxidationsmittel eingespeist wird. Alternativ kann das zusätzliche Oxidationsmittel auch in jeden der seriell angeordneten Reaktoren eingespeist werden.

In einer bevorzugten Ausführungsform des erfindungsgemäßen Verfahrens werden daher der Schritt b) und, falls erforderlich, der Schritt c) nach dem ersten Reaktor und bei mehr als zwei seriell angeordneten Reaktoren auch nach dem zweiten und jedem weiteren Reaktor durchgeführt.

Hinsichtlich der Wahl des zusätzlichen Oxidationsmittels unterliegt das erfindungsgemäße Verfahren keinen Beschränkungen. Es ist möglich, im gesamten Verfahren ausschließlich freien Sauerstoff oder Sauerstoff in gebundener Form, wie vorstehend definiert, als Oxidationsmittel einzusetzen. Alternativ ist es auch möglich, freien Sauerstoff und Sauerstoff in gebundener Form in jeweils unterschiedlichen Mengen in unterschiedlichen Schritten des erfindungsgemäßen Verfahrens einzusetzen. Gegenüber Sauerstoff in gebundener Form, beispielsweise als Salpetersäure, hat die Verwendung von freiem Sauerstoff als Oxidationsmittel den Vorteil, dass ein kostengünstiges Oxidationsmittel zum Einsatz kommt. Der Vorteil von Sauerstoff in gebundener Form besteht hingegen darin, dass er in der Regel ein höheres Oxidationspotential hat als freier Sauerstoff. Durch die Wahl der für die jeweiligen Verfahrensschritte geeigneten sauerstoffhaltigen Oxidationsmittel können die jeweiligen Vorteile der betreffenden Oxidationsmittel optimal genutzt werden. Die Verwendung von freiem Sauerstoff als Oxidationsmittel ist besonders dann von Vorteil, wenn es darum geht, große Mengen schwefelhaltiger Ausgangsverbindungen zu oxidieren. Dies ist typischerweise bei der Durchführung von Schritt a) im ersten von mindestens zwei Reaktoren der Fall. Der Einsatz von Sauerstoff in gebundener Form als Oxidationsmittel, bevorzugt Salpetersäure, erfolgt zweckmäßigerweise dann, wenn die Oxidationsreaktion vervollständigt werden muss. Dies ist typischerweise der Fall, wenn in Schritt c) des erfindungsgemäßen Verfahrens zusätzliches Oxidationsmittel zur Durchführung von Schritt a) in den zweiten oder jeden weiteren Reaktor einer Serie von Reaktoren eingespeist wird, oder wenn der Schritt a) in nur einem Reaktor durchgeführt wird.

In einer weiteren bevorzugten Ausführungsform des erfindungsgemäßen Verfahrens wird daher die Oxidation im ersten Reaktor mit freiem Sauerstoff durchgeführt, und bei mehr als zwei seriell angeordneten Reaktoren wird, falls erforderlich, Sauerstoff in gebundener Form in den zweiten und jeden weiteren Reaktor eingespeist.

Im Rahmen der vorliegenden Erfindung hat sich gezeigt, dass UV-Vis-Spektroskopie und gepulste amperometrische Detektion sowohl alleine als auch in Kombination miteinander dazu geeignet sind, die Herstellung von Alkansulfonsäure durch Oxidation von schwefelhaltigen Ausgangsverbindungen zu steuern. Durch die erfindungsgemäße Verwendung von UV-Vis-Spektroskopie und/oder gepulster amperometrischer Detektion werden erfindungsgemäß sowohl nicht umgesetzte schwefelhaltige Ausgangsverbindungen als auch Oxidationszwischenprodukte, die zu Niederschlägen von elementarem Schwefel und damit verbundenen Verstopfungen von Rohrleitung und Kolonnenköpfen führen können, bestimmt. Auf Basis dieser Bestimmung kann die zur Vervollständigung der Oxidationsreaktion erforderliche Menge zusätzlichen Oxidationsmittels in den oder die betreffenden Reaktoren zugeführt werden. Die Durchführung der Oxidation von schwefelhaltigen Ausgangsverbindungen kann unabhängig von der konkreten Ausgestaltung der Verfahrensführung maßgeblich mit Hilfe des Einsatzes von UV-Vis-Spektroskopie und/oder gepulster amperometrischer Detektion gesteuert werden.

Ein weiterer Gegenstand der vorliegenden Erfindung ist daher auch die Verwendung von UV-Vis-Spektroskopie und/der gepulster amperometrischer Detektion in der Herstellung von Alkansulfonsäuren.

In einer Ausführungsform der erfindungsgemäßen Verwendung erfolgt die Herstellung von Alkansulfonsäuren durch Oxidieren von schwefelhaltigen Ausgangsverbindungen, wobei die schwefelhaltigen Ausgangsverbindungen ausgewählt sind aus der Gruppe bestehend aus Alkylmercaptan, Dialkyldisulfid und Dialkylpolysulfid mit drei bis neun Schwefelatomen.

In einer bevorzugten Ausführungsform der erfindungsgemäßen Verwendung erfolgt die Herstellung von Alkansulfonsäuren durch Oxidieren von Methylmercaptan, Dimethyldisulfid und/oder Dimethylpolysulfid mit drei bis neun Schwefelatomen.

Das erfindungsgemäße Konzept, die Durchführung der Oxidation einer organischen Verbindung zur gewünschten Zielverbindung über die Bestimmung von Ausgangsverbindungen und Oxidationszwischenprodukten zu steuern, ist nicht auf die Oxidation von schwefelhaltigen Ausgangsverbindungen zu den entsprechenden Alkansulfonsäuren beschränkt. Vielmehr lässt sich dieses Konzept auch auf die Durchführung anderer Oxidationsreaktionen übertragen.

Ein weiterer Gegenstand der vorliegenden Erfindung ist daher auch eine Vorrichtung zur Durchführung von Oxidationsreaktionen, umfassend

i) mindestens einen Reaktor zur Oxidation mindestens einer Ausgangsverbindung,

ii) mindestens eine dem Reaktor nachgeschaltete Messeinrichtung zur Bestimmung von nicht oxidierten Ausgangsverbindungen und/oder Oxidationszwischenprodukten aus der Oxidation der Ausgangsverbindung zur gewünschten Zielverbindung im Reaktionsaustrag aus Schritt a), und

iii) mindestens eine mit der Messeinrichtung in Kontakt stehende Einrichtung zur Zuführung von zusätzlichem Oxidationsmittel in den mindestens einen Reaktor.

Auf die Anordnung der Komponenten i) bis iii) der erfindungsgemäßen Vorrichtung und die Betriebsweise dieser Komponenten treffen dieselben Aspekte zu wie auf die Anordnung und Betriebsweise der entsprechenden Komponenten im erfindungsgemäßen Verfahren, insbesondere bezüglich der Anzahl und Anordnung der Reaktoren sowie der Bestimmung von nicht oxidierten Ausgangsverbindungen und/oder Oxidationszwischenprodukten aus der Oxidation der Ausgangsverbindung zur gewünschten Zielverbindung und der Zuführung von zusätzlichem Oxidationsmittel.

In einer Ausführungsform weist die erfindungsgemäße Vorrichtung daher mindestens zwei seriell angeordnete Reaktoren auf.

In einer weiteren Ausführungsform weist die erfindungsgemäße Vorrichtung Messeinrichtungen zur Bestimmung von nicht oxidierten Ausgangsverbindungen und/oder von nicht oxidierten

Oxidationszwischenprodukten nach dem ersten Reaktor und bei mehr als zwei in Serie angeordneten Reaktoren auch nach dem zweiten und jedem weiteren Reaktor auf.

Hinsichtlich der Messeinrichtung zur Bestimmung von nicht umgesetzten Ausgangsverbindungen und/oder Oxidationszwischenprodukten unterliegt die erfindungsgemäße Vorrichtung keinen Beschränkungen. Vielmehr ist prinzipiell der Einsatz aller Messeinrichtungen möglich, die eine verlässliche Bestimmung von nicht umgesetzten Ausgangsverbindungen und/oder Oxidationszwischenprodukten in einem möglichst kleinen Zeitintervall erlaubt, damit ohne eine wesentliche Zeitverzögerung, vorzugsweise innerhalb weniger Sekunden nach der Bestimmung, zusätzliches Oxidationsmittel in den mindestens einen Reaktor zur Vervollständigung der Oxidationsreaktion zugeführt werden. Unter dieser Voraussetzung haben sich das UV-Vis-Spektrometer und der gepulste amperometrische Detektor als für die erfindungsgemäße Vorrichtung geeignete Messeinrichtungen erwiesen. Denn mit UV-Vis-Spektrometern und gepulsten amperometrischen Detektoren können innerhalb kurzer Zeit und ohne großen apparatetechnischen Aufwand nicht umgesetzte Ausgangsverbindungen und/oder Oxidationszwischenprodukte im Reaktionsaustrag aus Oxidationsreaktionen zuverlässig und mit hoher Genauigkeit bestimmt werden. Ob zur Bestimmung nur ein UV-Vis-Spektrometer, nur ein gepulster amperometrischer Detektor oder eine Kombination davon eingesetzt wird, richtet sich nach der jeweiligen Eignung der betreffenden Ausgangsverbindungen und den bei der Oxidation durchlaufenen Oxidationszwischenprodukten für die jeweiligen Bestimmungsmethoden.

In einer Ausführungsform der erfindungsgemäßen Vorrichtung ist die Messeinrichtung daher ein UV-Vis-Spektrometer und/oder ein amperometrischer Detektor, bevorzugt ein gepulster amperometrischer Detektor.

In einer bevorzugten Ausführungsform weist die erfindungsgemäße Vorrichtung ein UV/VIS-Spektrometer und einen amperometrischen Detektor nach dem ersten Reaktor und bei mehr als zwei seriell angeordneten Reaktoren auch dem zweiten und jedem weiteren Reaktor auf.

In einer zusätzlichen Ausführungsform weist die erfindungsgemäße Vorrichtung eine Einrichtung zur Einspeisung von freiem Sauerstoff in den ersten Reaktor auf, um die Oxidationsreaktion im ersten Reaktor durchführen zu können, und bei mehr als zwei seriell angeordneten Reaktoren auch Einrichtungen zur Einspeisung von Sauerstoff in gebundener Form in den zweiten oder jeden weiteren Reaktor, um die Oxidationsreaktion zum gewünschten Oxidationsprodukt zu vervollständigen.

Die folgenden Beispiele belegen die Eignung der gepulsten amperometrischen Detektion zur Bestimmung von Oxidationszwischenprodukten, insbesondere von Alkansulfonsäure-S-alkylestern wie Methansulfonsäure-S-methylester, in einer Alkansulfonsäure, insbesondere Methansulfonsäure, enthaltenden Matrix.

**Abbildungen:**

Figur 1:    Chromatogramm des Vergleichsbeispiels 1
Figur 2:    Chromatogramm des Vergleichsbeispiels 2
Figur 3:    Chromatogramm des Vergleichsbeispiels 3
Figur 4:    Chromatogramm des Vergleichsbeispiels 4
Figur 5:    Chromatogramm des Beispiels 1
Figur 6:    Chromatogramm des Beispiels 2
Figur 7:    Chromatogramm des Beispiels 3
Figur 8:    Chromatogramm des Beispiels 4
Figur 9:    $^1$H NMR-Spektrum des Vergleichsversuchs 5

**Beispiele:**

**I. Technische Ausstattung**

**1. Verwendete Geräte:**

Probenaufgabesystem Professional Sample Processor 858 (Nr. 2.858.0010 von Metrohm) ausgestattet mit einem Dosiersystem 800 Dosino 800 (Nr. 2.800.0010 von Metrohm).

Ionenchromatograph 882 Compact IC plus (Nr. 2.850.9110 von Metrohm) ausgestattet mit einem UV/VIS Detektor Vario 944 (Nr. 2.944.0010 von Metrohm), einem IC Amperometrischen Detektor (Nr. 2.850.9110 von Metrohm) und einer Druckanzeige.

Ionenchromatograph 883 Basic IC plus (2.883.0020 von Metrohm) ausgestattet mit einem Leitfähigkeitsdetektor in Form des Moduls iDetector (standardmäßige Ausstattung des Ionenchromatographen 883 Basic IC plus) und einer Druckanzeige.

In den Versuchen ist das erste Gerät das Probenaufgabesystem, gefolgt vom Ionenchromatograph, der mit einem entsprechenden Detektor ausgestattet ist.

**2. Chromatographie-Säulen:**

Im Ionenchromatograph 882 Compact IC plus wird eine Säule von Typ Phenomenex® Gemini® 5U C6-Phenyl 110A 250/4.6 verwendet. Alternativ kann auch eine Säule vom Typ ProntoSil 120-5-C18 AQ 150/4.0 (6.1008.100 von Metrohm) verwendet werden.

Im Ionenchromatograph 883 Basic IC Plus wurde ein Metrosep A Supp 1 Guard/4.6 als Vorsäule bzw. Guardsäule und ein Anionentrennsäule Metrosep A Supp 5 250/4.0 als Hauptsäule verwendet. Ferner wurde ein Metrohm Suppressor-Modul MSM als Kationentauscher verwendet.

**3. Eluenten:**

Eingesetzte Chemikalien:

- Reinstwasser mit einem Leitwiderstand von 18,2 MOhm und einem TOC-Wert von 5 ppb, wobei der TOC für organic organic carbon steht und die Summe des gesamten organischen Kohlenstoffs in einer Wasserprobe angibt (aus einer Reinstwasseranlage Milli-Q Advantage A10 mit Q-POC Entnahmestelle oder einer Millipore-Anlage),
- Methanol LiChrosolv HPLC Grade (high performance liquid chromatography) (1.06007 von Merck),
- Kaliumdihydrogenphosphat 99 %(1.04873 von Merck),
- Phosphorsäure 85 % (1.00573 von Merck), und
- Methansulfonsäure (471356 von Sigma Aldrich).

Der Eluent für den Ionenchromatograph 882 Compact IC plus setzte sich zusammen aus:

- 70 % Reinstwasser,
- 30 % Methanol,
- 10 % Kaliumdihydrogenphosphat, und
- 1,2 g Phosphorsäure.

Der Eluent für den Ionenchromatograph 883 Basic IC plus setzte sich zusammen aus:

- 100 % Reinstwasser,
- 3,2 mmol Natriumcarbonat, und
- 1,0 mmol Diyhdrogencarbonat.

**II. Amperometrische Detektion bei konstanter Spannung**

Zum Vergleich mit der gepulsten amperometrischen Detektion wurde Dimethyldisulfid (DMDS) in einer Probe aus der Herstellung von Methansulfonsäure (MSA) durch Oxidation von Dimethyldisulfid mittels amperometrischer Detektion bei konstanter Spannung bestimmt: Es wurde ein Analyt verwendet, der durch Auflösen von 2 Tropfen der Probe aus der Methansulfonsäure-Herstellung in 50 ml eines Gemisches aus Acetonitril und Wasser (Volumenverhältnis 30:70) bereitgestellt wurde (im Folgenden als Analyt V bezeichnet). Durchgeführt wurden die Messungen über einen Zeitraum von ungefähr einer Stunde mit einem Ionenchromatograph 882 Compact IC Plus 1 (2.850.9110 der Firma Metrohm), der mit einer Trennsäule ProntoSil 120-5-C18 AQ - 150/4.0 (6.1008.100 der Firma Metrohm), einem Metrosep RP2 Guard /3.5 (6.1011.030 der Firma Metrohm) und einem amperometrischen Detektor (2.850.9110 der Firma Metrohm) ausgestattet war. Dieser Detektor besitzt eine Messzelle vom Wall Jet Cell-Typ (6.5337.020 der Firma Metrohm), die ausgestattet ist mit einer Glaskohlenstoff-Arbeitselektrode (6.1257.220 der Firma Metrohm) von 3 mm Durchmesser, einer Silber/Silberchlorid-Referenzelektrode (6.1257.720 der Firma Metrohm) und einer Hilfselektrode (6.1247.000 der Firma Metrohm). Das Injektionsvolumen betrug 20 μl, und die Temperatur der Trennsäule befand sich bei ca. 25°C.

Im Anschluss wurden die Bestimmungen von Dimethyldisulfid miteinander verglichen, um Aussagen über die Reproduzierbarkeit und Verlässlichkeit der amperometrischen Bestimmung bei konstanter Spannung machen zu können. Hierfür wurden die Werte für die Fläche unter dem Peak für Dimethyldisulfid in den jeweiligen Chromatogrammen miteinander verglichen.

**Vergleichsbeispiel 1:**

Zum Zeitpunkt t = 0 min wurde der Analyt V in den Ionenchromatograph eingespritzt. Die Parameter der durchgeführten Ionenchromatographie waren:

Eluent: $MeOH : H_2O\,(3:7) + 2\,g/l\,KH_2PO_4 + 2\,g/l\,H_3PO_4$
Fluss: 1,2 ml/min
Druck: 173,1 bar
Aufnahmedauer: 20,7 min

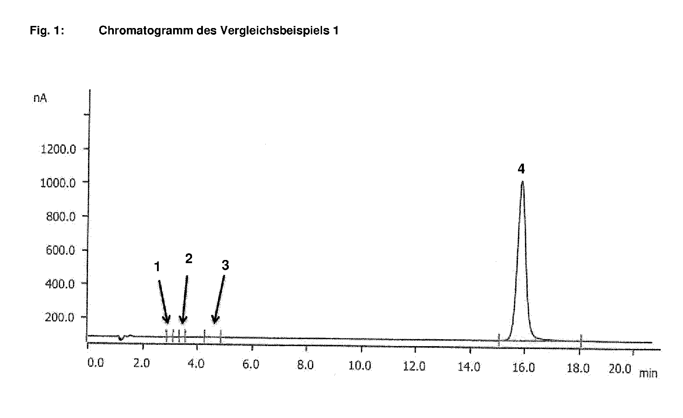

Die im Eluat dieser Ionenchromatographie bestimmten Komponenten sind in der Tabelle 1 aufgelistet und das Chromatogramm dieser Bestimmung ist in Abbildung 1 dargestellt. Die Nummern der Peaks in dieser Tabelle stimmen mit den entsprechend bezifferten Peaks im Chromatogramm der Abbildung 1 überein.

**Tabelle 1: Ergebnisse der Bestimmung in Vergleichsbeispiel 1**

| Peak-Nr. | Retentionszeit [min] | Fläche (nA) * min | Höhe [nA] | Komponente |
|---|---|---|---|---|
| 1 | 2,997 | 0,0253 | 0,176 | Bromid |
| 2 | 3,457 | 0,0163 | 0,148 | Nitrat |
| 3 | 4,415 | 0,4227 | 3,483 | Phosphat |
| 4 | 15,835 | 352,7363 | 958,275 | DMDS |

**Vergleichsbeispiel 2:**

Zum Zeitpunkt t = 22 min wurde der Analyt V in den Ionenchromatograph eingespritzt. Die Parameter der durchgeführten Ionenchromatographie waren:

Eluent: $MeOH : H_2O\,(3:7) + 2\,g/l\,KH_2PO_4 + 2\,g/l\,H_3PO_4$
Fluss: 1,2 ml/min
Druck: 170,8 bar
Aufnahmedauer: 18,5 min

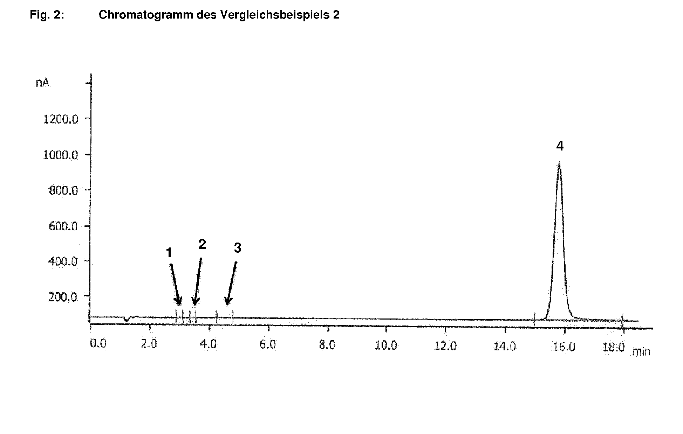

Die im Eluat dieser Ionenchromatographie bestimmten Komponenten sind in der Tabelle 2 aufgelistet und das Chromatogramm dieser Bestimmung ist in Abbildung 2 dargestellt. Die Nummern der Peaks in dieser Tabelle stimmen mit den entsprechend bezifferten Peaks im Chromatogramm der Abbildung 2 überein.

**Tabelle 2: Ergebnisse der Bestimmung in Vergleichsbeispiel 2**

| Peak-Nr. | Retentionszeit [min] | Fläche (nA) * min | Höhe [nA] | Komponente |
|---|---|---|---|---|
| 1 | 2,988 | 0,0230 | 0,163 | Bromid |
| 2 | 3,455 | 0,0140 | 0,149 | Nitrat |
| 3 | 4,405 | 0,3272 | 2,717 | Phosphat |
| 4 | 15,752 | 321,3513 | 891,404 | DMDS |

Bei Verwendung eines identischen Analyten wurde mit der amperometrischen Bestimmung bei konstanter Spannung bereits 22 Minuten nach der ersten Messung (Vergleichsbeispiel 1) ein um 8,9% geringerer Flächen-Wert für die Bestimmung von Dimethyldisulfid in Methansulfonsäure erhalten.

**Vergleichsbeispiel 3:**

Zum Zeitpunkt t = 42 min wurde der Analyt V in den Ionenchromatograph eingespeist. Die Parameter der durchgeführten Ionenchromatographie waren:

Eluent: $MeOH : H_2O\,(3:7) + 2\,g/l\,KH_2PO_4 + 2\,g/l\,H_3PO_4$
Fluss: 1,2 ml/min
Druck: 173,1 bar

(fortgesetzt)

Aufnahmedauer: 20,7 min

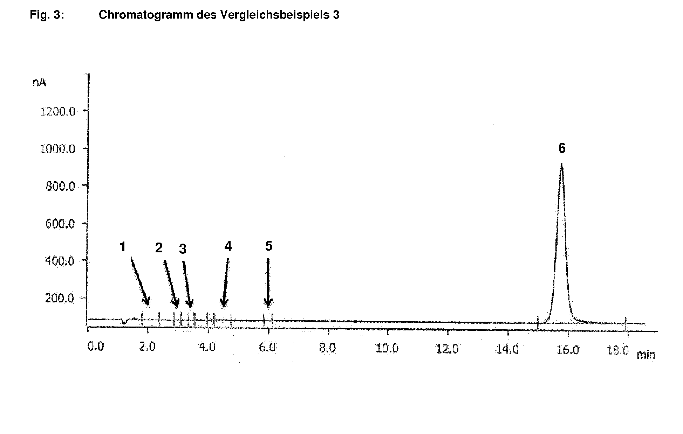

Die im Eluat dieser Ionenchromatographie bestimmten Komponenten sind in der Tabelle 3 aufgelistet und das Chromatogramm dieser Bestimmung ist in Abbildung 3 dargestellt. Die Nummern der Peaks in dieser Tabelle stimmen mit den entsprechend bezifferten Peaks im Chromatogramm der Abbildung 3 überein.

Tabelle 3: Ergebnisse der Bestimmung in Vergleichsbeispiel 3

| Peak-Nr. | Retentionszeit [min] | Fläche (nA) * min | Höhe [nA] | Komponente |
|---|---|---|---|---|
| 1 | 1,908 | 0,0337 | 0,175 | MSA |
| 2 | 2,992 | 0,0234 | 0,175 | Bromid |
| 3 | 3,458 | 0,0148 | 0,154 | Nitrat |
| 4 | 4,403 | 0,2953 | 2,442 | Phosphat |
| 5 | 6,002 | 0,0066 | 0,030 | MMTS |
| 6 | 15,705 | 303,7108 | 853,682 | DMDS |

Bereits 42 Minuten nach der ersten Messung (Vergleichsbeispiel 1) wurde mittels amperometrischer Bestimmung bei konstanter Spannung ein um 13,9 % geringerer Flächen-Wert für Dimethyldisulfid erhalten.

**Vergleichsbeispiel 4:**

Zum Zeitpunkt t = 62 min wurde der Analyt V in den Ionenchromatograph eingespritzt. Die Parameter der durchgeführten Ionenchromatographie waren:

Eluent: $MeOH : H_2O$ (3:7) + 2 g/l $KH_2PO_4$ + 2 g/l $H_3PO_4$
Fluss: 1,2 ml/min
Druck: 168,0 bar
Aufnahmedauer: 25,0 min

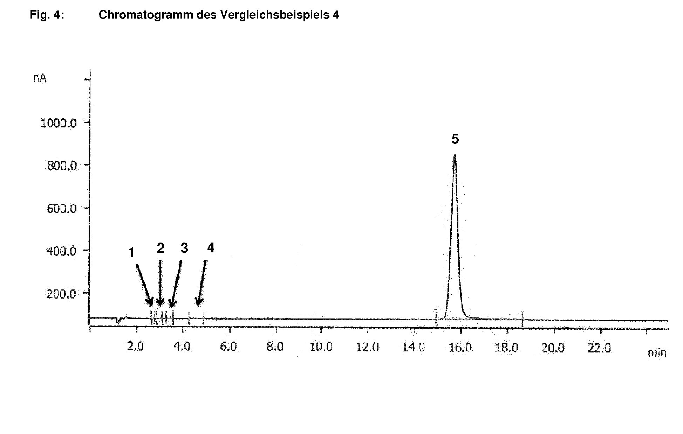

Die im Eluat dieser Ionenchromatographie bestimmten Komponenten sind in der Tabelle 4 aufgelistet und das Chromatogramm dieser Bestimmung ist in Abbildung 4 dargestellt. Die Nummern der Peaks in dieser Tabelle stimmen mit den entsprechend bezifferten Peaks im Chromatogramm der Abbildung 4 überein.

Tabelle 4: Ergebnisse der Bestimmung in Vergleichsbeispiel 4

| Peak-Nr. | Retentionszeit [min] | Fläche (nA) * min | Höhe [nA] | Komponente |
|---|---|---|---|---|
| 1 | 2,720 | 0,0037 | 0,059 | Nitrit |
| 2 | 3,000 | 0,0232 | 0,150 | Bromid |
| 3 | 3,468 | 0,0191 | 0,154 | Nitrat |
| 4 | 4,402 | 0,2589 | 2,106 | Phosphat |
| 5 | 15,682 | 275,3675 | 771,120 | DMDS |

62 Minuten nach der ersten Messung (Vergleichsbeispiel 1) lag der Flächen-Wert für die Bestimmung von Dimethyldisulfid sogar um 21,9 % unter dem Ausgangswert.
Die Vergleichsversuche 1 bis 4 zeigen, dass eine amperometrische Detektion mit konstanter Spannung grundsätzlich nicht geeignet ist für eine reproduzierbare und verlässliche Bestimmung von Dialkyldisulfiden in Alkansulfonsäuren und insbesondere von Dimethyldisulfiden in Methansulfonsäure.

**III. Gepulste amperometrische Detektion**

Mittels gepulster amperometrischer Detektion wurde über einen Zeitraum von mehr als einer Stunde Dimethyldisulfid in einer Probe aus der Herstellung von Methansulfonsäure durch Oxidation von Dimethyldisulfid bestimmt.

Hierfür wurde ein Analyt verwendet, der durch Auflösen von 3 Tropfen der Probe aus der Methansulfonsäure-Herstellung in 100 ml eines Gemisches aus Acetonitril und Wasser (Volumenverhältnis 30:70) bereitgestellt wurde (im Folgenden als Analyt B bezeichnet).

Es wurde dieselbe instrumentelle Anordnung wie bei der amperometrischen Detektion mit konstanter Spannung verwendet. Das Injektionsvolumen betrug 20 µl, und die Temperatur der Trennsäule befand sich bei ca. 25°C.

Das Elektrooxidationspotential hatte einen Wert von 1,15 V und eine Dauer von 300 ms, wobei die Messdauer 100 ms betrug. Das Reinigungspotential hatte einen Wert von 1,5 V und eine Dauer von 50 ms und das Konditionierungspotential hatte einen Wert von 0,1 V und eine Dauer von 200 ms. Die Gesamtdauer eines Messzyklus betrug somit 550 ms.

**Beispiel 1:**

Zum Zeitpunkt t = 0 min wurde der Analyt B in den Ionenchromatograph eingespritzt. Die Parameter der durchgeführten Ionenchromatographie waren:

| | |
|---|---|
| Eluent: | MeOH : $H_2O$ (3:7) + 4,2 g/l $KH_2PO_4$ + 0,2 g/l $H_3PO_4$ |
| Fluss: | 1,2 ml/min |
| Druck: | 160,1 bar |
| Aufnahmedauer: | 25,0 min |

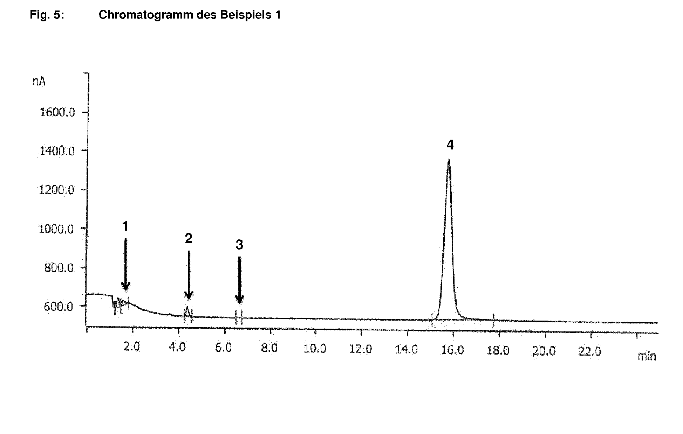

Die im Eluat dieser Ionenchromatographie bestimmten Komponenten sind in der Tabelle 5 aufgelistet und das Chromatogramm dieser Bestimmung ist in Abbildung 5 dargestellt. Die Nummern der Peaks in dieser Tabelle stimmen mit den entsprechend bezifferten Peaks im Chromatogramm der Abbildung 5 überein.

**Tabelle 5: Ergebnisse der Bestimmung in Beispiel 1**

| Peak-Nr. | Retentionszeit [min] | Fläche (nA) * min | Höhe [nA] | Komponente |
|---|---|---|---|---|
| 1 | 1,631 | 3,7303 | 17,486 | Fluorid |
| 2 | 4,390 | 5,6792 | 48,794 | Phosphat |
| 3 | 6,644 | 0,2156 | 1,330 | MMTS |
| 4 | 15,716 | 333,7089 | 771,120 | DMDS |

**Beispiel 2:**

Zum Zeitpunkt t = 42 min wurde der Analyt B in den Ionenchromatograph eingespritzt. Die Parameter der durchgeführten Ionenchromatographie waren:

| | |
|---|---|
| Eluent: | MeOH : $H_2O$ (3:7) + 4,2 g/l $KH_2PO_4$ + 0,2 g/l $H_3PO_4$ |
| Fluss: | 1,2 ml/min |
| Druck: | 161,2 bar |
| Aufnahmedauer: | 21,7 min |

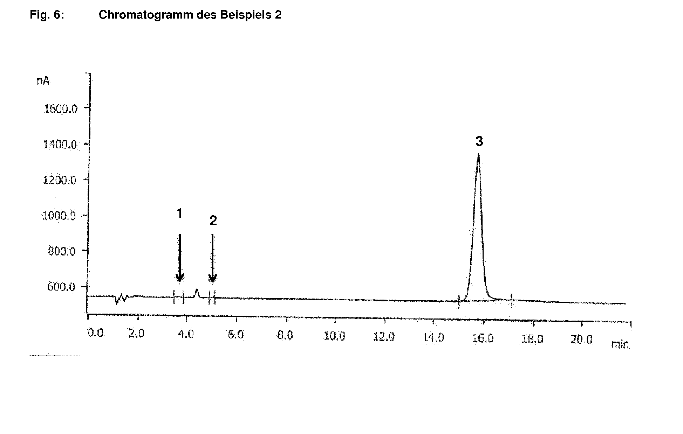

Die im Eluat dieser Ionenchromatographie bestimmten Komponenten sind in der Tabelle 6 aufgelistet und das Chromatogramm dieser Bestimmung ist in Abbildung 6 dargestellt. Die Nummern der Peaks in dieser Tabelle stimmen mit den entsprechend bezifferten Peaks im Chromatogramm der Abbildung 6 überein.

**Tabelle 6: Ergebnisse der Bestimmung in Beispiel 2**

| Peak-Nr. | Retentionszeit [min] | Fläche (nA) * min | Höhe [nA] | Komponente |
|---|---|---|---|---|
| 1 | 3,620 | 0,7375 | 6,608 | Nitrat |

(fortgesetzt)

| Peak-Nr. | Retentionszeit [min] | Fläche (nA) * min | Höhe [nA] | Komponente |
|---|---|---|---|---|
| 2 | 4,985 | 0,2138 | 1,922 | Phosphat |
| 3 | 15,689 | 327,0947 | 825,014 | DMDS |

**Beispiel 3:**

Zum Zeitpunkt t = 2,5 h wurde der Analyt B in den Ionenchromatograph eingespritzt. Die Parameter der durchgeführten Ionenchromatographie waren:

Eluent:          MeOH : $H_2O$ (3:7) + 4,2 g/l $KH_2PO_4$ + 0,2 g/l $H_3PO_4$
Fluss:           1,2 ml/min
Druck:           161,2 bar
Aufnahmedauer:   21,7 min

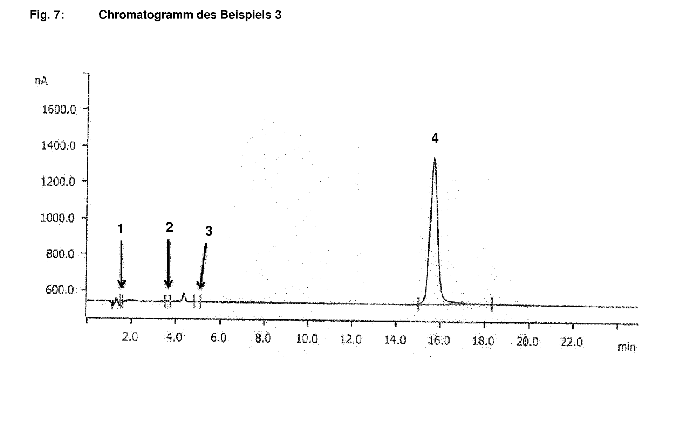

Die im Eluat dieser Ionenchromatographie bestimmten Komponenten sind in der Tabelle 7 aufgelistet und das Chromatogramm dieser Bestimmung ist in Abbildung 7 dargestellt. Die Nummern der Peaks in dieser Tabelle stimmen mit den entsprechend bezifferten Peaks im Chromatogramm der Abbildung 7 überein.

**Tabelle 7: Ergebnisse der Bestimmung in Beispiel 3**

| Peak-Nr. | Retentionszeit [min] | Fläche (nA) * min | Höhe [nA] | Komponente |
|---|---|---|---|---|
| 1 | 1,576 | 0,4106 | 8,942 | Fluorid |
| 2 | 3,629 | 0,4906 | 4,236 | Nitrat |
| 3 | 4,994 | 0,2911 | 1,955 | Phosphat |
| 4 | 15,652 | 332,5320 | 811,847 | DMDS |

**Diskussion der Messergebnisse:**

Zur Beurteilung der Genauigkeit der gepulsten amperometrischen Detektion wurden die in den Beispielen 1 bis 3 bestimmten Werte für die Fläche unter den Peaks für das Dimethyldisulfid herangezogen. Denn dieser Wert ist ein Anhaltspunkt für die Konzentration des zu bestimmenden Dimethyldisulfids.
Die in den Beispielen 1 und 2 bestimmten Flächenwerte wichen lediglich um 1,98 % voneinander ab. Die Abweichung der Flächenwerte für die Beispiele 1 und 3, die zeitlich am weitesten auseinanderliegen, betrug sogar nur 0,35 %. Da die in den Beispielen 1 und 3 ermittelten Werte nahezu identisch waren, erlaubt die gepulste amperometrische Detektion also eine zuverlässige Bestimmung des Dimethyldisulfids. Die im Beispiel 2 aufgetretene Abweichung ist daher nicht auf eine eventuelle mangelnde Reproduzierbarkeit der Messergebnisse sondern auf einen Messfehler zurückzuführen.
Die in diesem Beispiel ermittelte Dimethyldisulfid-Konzentration war identisch mit der aus Beispiel 1. Zudem betrug die Abweichung von dem in Beispiel 1 ermittelten Flächen-Wert lediglich 0,35%, was noch deutlich unter dem bereits geringen Messfehler des Beispiels 2 lag. Gegenüber den Vergleichsbeispielen 2 bis 4 waren die Abweichungen für den Flächen-Wert in den Beispiele 2 und 3 erheblich geringer, und dies zudem über einen Zeitraum, der mehr als doppelt so lang war wie die gesamte Messdauer in den Vergleichsbeispielen. Folglich stellt die gepulste amperometrische Detektion eine zuverlässige und reproduzierbare Bestimmung von Dimethyldisulfid in Methansulfonsäure dar.

**IV. Vergleich von Ionenchromatographie und gepulster Amperometrie mit NMR**

Nachdem gezeigt wurde, dass die gepulste amperometrische Detektion eine geeignete Methode zur reproduzierbaren und verlässlichen Bestimmung von Dialkyldisulfiden in Alkansulfonsäuren, insbesondere von Dimethyldisulfid in Methansulfonsäure, ist, wurde die Genauigkeit dieser Methode mit der Kernspinresonanz verglichen.

**Beispiel 4:**

Es wurde dieselbe instrumentelle Anordnung wie bei der amperometrischen Detektion bei konstanter Spannung verwendet. Als Analyt wurde ein Gemisch aus 3 Tropfen einer Probe aus der Herstellung von Methansulfonsäure durch Oxidation von Dimethyldisulfid in 100 ml eines Gemisches aus Acetonitril und Wasser (30:70 Vv) verwendet. Das in den Ionenchromatograph eingespritzte Volumen betrug 20 $\mu$l, und die Temperatur der Trennsäule lag bei ca. 25°C.

| | |
|---|---|
| Eluent: | MeOH : $H_2O$ (3:7) + 4,2 g/l $KH_2PO_4$ + 0,2 g/l $H_3PO_4$ |
| Fluss: | 1,2 ml/min |
| Druck: | 162,3 bar |
| Aufnahmedauer: | 25,0 min |

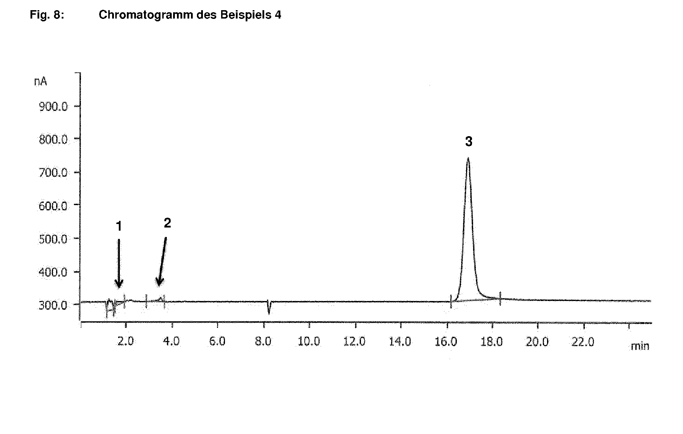

Die im Eluat dieser Ionenchromatographie bestimmten Komponenten sind in der Tabelle 8 aufgelistet und das Chromatogramm dieser Bestimmung ist in Abbildung 8 dargestellt. Die Nummern der Peaks in dieser Tabelle stimmen mit den entsprechend bezifferten Peaks im Chromatogramm der Abbildung 8 überein.

**Tabelle 8: Ergebnisse der Bestimmung in Beispiel 5**

| Peak-Nr. | Retentions-zeit [min] | Fläche (nA) * min | Höhe [nA] | Konzentration [Gew.-%] | Komponente |
|---|---|---|---|---|---|
| 1 | 1,686 | 2,3189 | 8,449 | - | MSA |
| 2 | 3,510 | 2,5620 | 12,960 | - | Nitrat |
| 3 | 15,926 | 190,6092 | 430,759 | 2,567 | DMDS |

**Vergleichsbeispiel 5:**

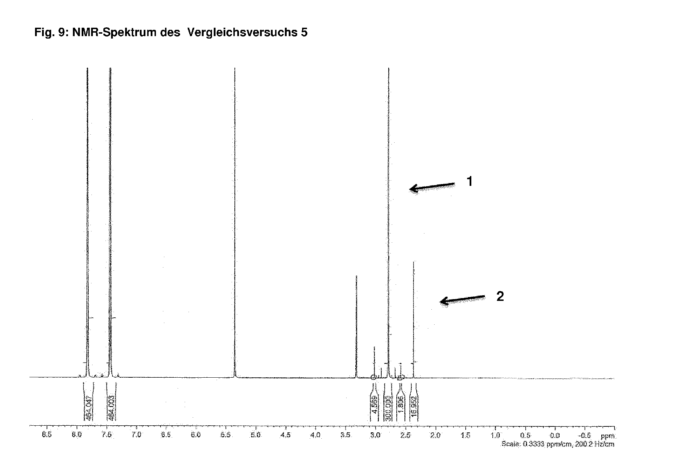

Dimethyldisulfid in Methansulfonsäure wurde auch mittels der Kernspinresonanz bestimmt. Der für diese Bestimmung verwendete Analyst setzte sich aus 26,95 mg einer Probe aus der Herstellung von Methansulfonsäure durch Oxidation von Dimethyldisulfid und 39,16 mg Naphthalin zusammen, wobei die letztgenannte Verbindung auf Grund ihres inerten Verhaltens gegenüber Dimethyldisulfid und Methansulfonsäure als Lösungsmittel diente. Die Kernspinresonanz-Messung wurde mit einem 600 MHz-Spektrometer Bruker Avance (III) der Firma Bruker Niospin, ausgerüstet mit einem 600 MHz-Magnetsystem Bruker Ascend der Firma Bruker Biospin und einem Prodigy cryo probe Probenkopf sowie unter Verwendung von MeOD als deuteriertem Lösungsmittel, durchgeführt. Die Messdauer betrug 20 Minuten. Das Protonenspektrum dieser Messung ist in Abbildung 9 dargestellt, und die aus diesem Spektrum erhaltenen Ergebnisse sind in der Tabelle 9 zusammengefasst.

Die Kernspinresonanz erlaubt die Bestimmung von Dimethyldisulfid mit einer Genauigkeit von einer Nachkommastelle bzw. 0,1 Gew.-%, während die gepulste amperometrische Detektion eine Bestimmung von Dimethyldisulfid bis auf drei Nachkommastellen bzw. 0,001 Gew.-% ermöglicht, was eine um den Faktor 100 größere Präzision darstellt.

Die in den Beispielen 1 bis 5 und im Vergleichsbeispiel 5 bestimmte Dimethyldisulfid-Konzentration bezieht sich immer auf die Konzentration dieser Komponente im jeweiligen Analyt. Da dieser Analyt aber eine Verdünnung der Probe aus der Methansulfonsäure-Herstellung darstellte, war die tatsächliche Konzentration des Dimethyldisulfids in dieser Probe um den Faktor der Verdünnung höher. Folglich machten sich bei konzentrierteren Proben die Unterschiede zwischen NMR-Analytik und gepulster amperometrische Detektion stärker bemerkbar. Auf Grund ihrer gegenüber der NMR-Analytik um den Faktor 100 besseren Genauigkeit ist die gepulste amperometrische Detektion daher die Methode der Wahl zur Bestimmung eines Dialkyldisulfids in einer Alkansulfonsäure.

**Tabelle 9: Ergebnisse der Bestimmung in Vergleichsbeispiel 5**

| Peak-Nr. | Komponente | Integral | Teiler | korrigiertes Integral | Verhältnis [Mol-%] | Stoffmenge [mMol] | molare Masse [g/mol] | Masse [mg] | Gehalt [Gew.-%] |
|---|---|---|---|---|---|---|---|---|---|
| | Naphthalin | 464,047 | 4 | 116,012 | 53,0 | 0,305 | 128,16 | 39,07 | - |
| 1 | MSA | 300,000 | 3 | 100,000 | 45,7 | 0,263 | 96,11 | 25,26 | 93,7 |
| 2 | DMDS | 16,952 | 6 | 2,825 | 1,3 | 0,007 | 94,20 | 0,70 | 2,6 |

**V. Vergleich des gepulsten amperometrischen Detektors mit einem UV-Detektor**

Die höhere Empfindlichkeit des (gepulsten) amperometrischen Detektors für ein Dialkyldisulfid in einer Alkansulfonsäure wurde durch eine Messreihe mit einer Verdünnungsreihe von Dimethyldisulfid in Methansulfonsäure gezeigt.

**1. Probenvorbereitung:**

Die Proben setzten sich aus den Einwaagen gemäß der Tabelle 10 zusammen, die auf einer Analysenwaage mit vier Nachkommastellen eingewogen wurden. Für eine Kalibrierung im ppm-Bereich wurde ein Tropfen der Probe auf 100 g Reinstwasser eingewogen. Eine flüssige Probe wurde in ein Probenfläschchen (Rotilabor) mit einem Volumen von 4 ml gegeben und im Anschluss mit einer Schraubkappe mitsamt Dichtung verschlossen. Die Probe wurde dann in das Probenrack des 858 Professional Sample Processors gestellt. Die weitere Verdünnung der Probe wurde mit dem Probensampler vorgenommen. Dies erfolgte in einem Mischbehälter mit Magnetrührer, wobei die Probe auf ein Verhältnis von 1:100 verdünnt wurde. Im Anschluss wurde die Probe in eine oder mehrere Probenschleifen unterschiedlicher Länge gepumpt. Bei einem Ionenchromatograph 882 Compact IC plus, der mit einer Säule des Typs Gemini 5U C6-Phenyl 110A 250/4.6 ausgestattet war, wies die Probenschleife eine Länge von 20 $\mu$l auf. Danach folgten Reinigungsschritte des Mischungsbehälters für die nächste Analyse.

**2. Abweichungen/Fehler:**

Eine Abweichung von ca. 2 % ist im Rahmen der Genauigkeit der Ionenchromatographie. Für die Bestimmung von Dimethyldisulfid waren Kalibrierungen in der Größenordnung von 50 ppm möglich, und für die Bestimmung von Methansulfonsäure-S-methylester, auch bekannt als Methylmethanthiosulfonat (MMTS), sind Kalibrierungen in der Größenordnung von 100 ppm möglich. Bei noch niedrigeren Konzentrationen wurden die Fehler deutlich größer als die akzeptable Abweichung von 2 %.

**3. Ergebnisse**

Es wurde ein Ionenchromatograph 882 Compact IC plus (Nr. 2.850.9110 von Metrohm) verwendet, der ausgestattet war mit einem UV/VIS Detektor Vario 944 (Nr. 2.944.0010 von Metrohm), einem IC Amperometrischen Detektor (Nr. 2.850.9110 von Metrohm) und einer Druckanzeige, die dazu diente einen konstanten Druck bei der Spektrenaufnahme nachverfolgen zu können.
Es wurden 4 Versuche mit den in der Tabelle 10 angegebenen Eingaben für die jeweiligen Analyten durchgeführt.

| Versuch | Kompo-nente | Einwaage | Sollwert % | Ist-Wert % | Abweichung % | Detektor |
|---------|-------------|----------|------------|------------|--------------|----------|
| 1 | MSA | 45,77 | 90,05 | 90 | 0,06 | LF |
| | MMTS | 1,95 | 3,84 | 3,849 | 0,12 | UV 210 |
| | DMDS | 3,10 | 6,10 | 6,108 | 0,08 | AD |
| | | | | 6,109 | 0,10 | UV 210 |
| 2 | MSA | 47,56 | 95,00 | 94,33 | -0,07 | LF |
| | MMTS | 0,99 | 1,98 | 1,941 | -1,75 | UV 210 |
| | DMDS | 1,52 | 3,03 | 3,011 | -0,57 | AD |
| | | | | 3,007 | -0,70 | UV 210 |
| 3 | MSA | 49,14 | 97,98 | 97,79 | -0,19 | LF |
| | MMTS | 0,5179 | 1,0320 | 1,079 | 4,56 | UV 210 |
| | DMDS | 0,4976 | 0,9921 | 0,996 | 0,40 | AD |
| | | | | 1 | 0,80 | UV 210 |
| 4 | MSA | 49,66 | 99,28 | 99,40 | 0,13 | LF |
| | MMTS | 0,1108 | 0,2214 | 0,19 | -14,19 | UV 210 |
| | DMDS | 0,2519 | 0,5034 | 0,518 | 2,89 | AD |
| | | | | 0,521 | 3,49 | UV 210 |

**Tabelle 10:** Zusammenstellung der Analyten und der Messergebnisse

Die in der Tabelle 10 zusammengefassten Messergebnisse zeigen, dass ein (gepulster) amperometrischer Detektor einem UV/VIS-Spektrometer hinsichtlich der Genauigkeit in der Bestimmung von Dimethyldisulfid überlegen ist. Diese höhere Genauigkeit der (gepulsten) Amperometrie in der Bestimmung von Dimethyldisulfid nimmt mit zunehmender Verdünnung des Dimethyldisulfids in der Methansulfonsäure sogar noch weiter zu.

**Patentansprüche**

1. Verfahren zur Herstellung einer Alkansulfonsäure, umfassend die Schritte

   d) Oxidieren mindestens einer schwefelhaltigen Ausgangsverbindung mit einem Oxidationsmittel zur korrespondierenden Alkansulfonsäure, wobei die schwefelhaltige Ausgangsverbindung ausgewählt ist aus der Gruppe bestehend aus Alkylmercaptan, Dialkyldisulfid und Dialkylpolysulfid mit drei bis neun Schwefelatomen,
   e) Bestimmen mindestens einer schwefelhaltigen Ausgangsverbindung und/oder mindestens eines Oxidationszwischenproduktes aus der Oxidation der schwefelhaltigen Ausgangsverbindung zur korrespondierenden Alkansulfonsäure im Reaktionsaustrag von Schritt a), und
   f) Zuführen von zusätzlichem Oxidationsmittel in Schritt a) zur Vervollständigung der Oxidationsreaktion, wenn in Schritt b) eine schwefelhaltige Ausgangsverbindung und/oder ein Oxidationszwischenprodukt aus der Oxidation der schwefelhaltigen Ausgangsverbindung zur korrespondierenden Alkansulfonsäure bestimmt wurden.

2. Verfahren gemäß Anspruch 1, wobei in Schritt b) ein Alkansulfonsäure-S-alkylester als Oxidationszwischenprodukt bestimmt wird.

3. Verfahren gemäß Anspruch 1 oder 2, wobei die Bestimmung des Oxidationszwischenproduktes in Schritt b) mittels UV-Vis-Spektroskopie erfolgt.

4. Verfahren gemäß einem beliebigen der Ansprüche 1 bis 3, wobei die Bestimmung der schwefelhaltigen Ausgangsverbindung in Schritt b) mittels gepulster amperometrischer Detektion erfolgt.

5. Verfahren gemäß einem beliebigen der Ansprüche 1 bis 4, wobei in Schritt b) ein Dialkyldisulfid und/oder ein Dialkylpolysulfid als schwefelhaltige Ausgangsverbindung bestimmt wird.

6. Verfahren gemäß einem beliebigen der Ansprüche 1 bis 5, wobei die Bestimmung von Oxidationszwischenprodukten und die Bestimmung von schwefelhaltigen Ausgangsverbindungen unabhängig voneinander in separaten Proben aus dem Reaktionsaustrag aus Schritt a) erfolgen.

7. Verfahren gemäß einem beliebigen der Ansprüche 1 bis 6, zusätzlich umfassend den dem Schritt b) vorgelagerten Schritt der chromatographischen Trennung einer Probe aus dem Reaktionsaustrag aus Schritt a).

8. Verfahren gemäß einem der Ansprüche 1 bis 7, wobei der Schritt a) mit freiem Sauerstoff und/oder mit Sauerstoff in gebundener Form durchgeführt wird.

9. Verfahren gemäß Anspruch einem beliebigen der Ansprüche 1 bis 8, wobei Schritt a) in mindestens einem Reaktor durchgeführt wird.

10. Verfahren gemäß Anspruch 9, wobei der Schritt a) in mindestens zwei seriell angeordneten Reaktoren durchgeführt wird.

11. Verfahren gemäß Anspruch 9 oder 10, wobei der Schritt b) und, falls erforderlich, der Schritt c) nach dem ersten Reaktor und bei mehr als zwei in Serie angeordneten Reaktoren auch nach dem zweiten und jedem weiteren Reaktor durchgeführt wird.

12. Verfahren gemäß Anspruch 10 oder 11, wobei die Oxidation im ersten Reaktor mit freiem Sauerstoff durchgeführt wird, und bei mehr als zwei in Serie angeordneten Reaktoren, falls erforderlich, Sauerstoff in gebundener Form in den zweiten und jeden weiteren Reaktor eingespeist wird.

13. Verwendung von UV-Vis-Spektroskopie und/oder gepulster amperometrischer Detektion in der Herstellung von Alkansulfonsäuren.

**14.** Vorrichtung zur Durchführung von Oxidationsreaktionen, umfassend

i) mindestens einen Reaktor zur Oxidation mindestens einer Ausgangsverbindung,
ii) mindestens eine dem Reaktor nachgeschaltete Messeinrichtung zur Bestimmung von nicht oxidierten Ausgangsverbindungen und/oder von Oxidationszwischenprodukten aus der Oxidation der Ausgangsverbindung zur gewünschten Zielverbindung im Reaktionsaustrag aus Schritt a), und
iii) eine mit der mindestens einen Messeinrichtung in Kontakt stehende Einrichtung zur Zuführung von zusätzlichem Oxidationsmittel in den Reaktor.

**15.** Vorrichtung gemäß Anspruch 14, wobei die Messeinrichtung ein UV-Vis-Spektrometer und/oder ein amperometrischer Detektor ist.

Fig. 1: Chromatogramm des Vergleichsbeispiels 1

Fig. 2:      Chromatogramm des Vergleichsbeispiels 2

Fig. 3:      Chromatogramm des Vergleichsbeispiels 3

Fig. 4:    Chromatogramm des Vergleichsbeispiels 4

Fig. 5:      Chromatogramm des Beispiels 1

Fig. 6:     Chromatogramm des Beispiels 2

Fig. 7: Chromatogramm des Beispiels 3

Fig. 8: Chromatogramm des Beispiels 4

Fig. 9: NMR-Spektrum des Vergleichsversuchs 5

Europäisches
Patentamt

European
Patent Office

Office européen
des brevets

**EUROPÄISCHER RECHERCHENBERICHT**

Nummer der Anmeldung

EP 15 19 7066

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2 489 318 A (PROELL WAYNE A) 29. November 1949 (1949-11-29) | 1-15 | INV. C07C303/16 |
| Y | * Anspruch 1 * | 1-15 | |
| Y | US 6 066 760 A (SCHON STEVEN GABRIEL [US]) 23. Mai 2000 (2000-05-23) * Abbildung 1 * * Beispiel 2b * | 1-15 | |
| Y | US 4 987 250 A (MCGEE DENNIS E [US] ET AL) 22. Januar 1991 (1991-01-22) * Spalte 10, Zeile 37 * * Spalte 7, Zeile 55 - Spalte 8, Zeile 11 * | 1-15 | |
| Y | INNOCENZO G. CASELLA ET AL: "Amperometric detection of sulfur-containing compounds in alkaline media", THE ANALYST, Bd. 127, Nr. 5, 2. April 2002 (2002-04-02) , Seiten 647-652, XP055224078, GB ISSN: 0003-2654, DOI: 10.1039/b111080m * Seite 647, linke spalte, letzter Absatz * | 1-15 | |
| Y | Antoaneta Pavlova ET AL: "Petroleum & Coal SULFUR COMPOUNDS IN PETROLEUM HYDROCARBON STREAMS", , 1. Januar 2012 (2012-01-01), XP055269474, Gefunden im Internet: URL:http://www.vurup.sk/sites/default/file s/downloads/pc_1_2012_pavlova_146.pdf [gefunden am 2016-04-28] * Zusammenfassung * | 1-15 | RECHERCHIERTE SACHGEBIETE (IPC) C07C |

Der vorliegende Recherchenbericht wurde für alle Patentansprüche erstellt

| Recherchenort | Abschlußdatum der Recherche | Prüfer |
|---|---|---|
| München | 2. Mai 2016 | Panday, Narendra |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 15 19 7066

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

02-05-2016

| Im Recherchenbericht angeführtes Patentdokument | Datum der Veröffentlichung | Mitglied(er) der Patentfamilie | | Datum der Veröffentlichung |
|---|---|---|---|---|
| US 2489318 A | 29-11-1949 | KEINE | | |
| US 6066760 A | 23-05-2000 | AT | 173465 T | 15-12-1998 |
| | | BR | 9501407 A | 27-02-1996 |
| | | CA | 2145327 A1 | 01-10-1995 |
| | | CN | 1113231 A | 13-12-1995 |
| | | DE | 69506022 D1 | 24-12-1998 |
| | | EP | 0675106 A1 | 04-10-1995 |
| | | JP | H0841010 A | 13-02-1996 |
| | | PL | 307953 A1 | 02-10-1995 |
| | | US | 6066760 A | 23-05-2000 |
| US 4987250 A | 22-01-1991 | KEINE | | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9834914 A1
- US 2433395 A
- US 2433396 A
- US 2498318 A
- US 2502618 A
- US 2505910 A
- US 2697722 A
- US 2727920 A
- WO 0031027 A1
- CN 101648892 A
- WO 0031027 A

**In der Beschreibung aufgeführte Nicht-Patentliteratur**

- **J. FRANK.** *Chimia,* 1981, vol. 35, 24
- **P. T. KISSINGER ; C.S. BRUNETT ; K. BRATIN ; J.R. RICE.** *Spec. Publ.,* 1979, vol. 519, 705
- **S. YAO ; A. MEYER ; G. HENZE ; FRESENIUS.** *J. Anal. Chem.,* 1991, vol. 339, 207
- **TERASHIMA et al.** *Analytical Chemistry,* 01. April 2003, vol. 75 (7), 1564-1572